# EUROPEAN PATENT APPLICATION

(11) **EP 4 659 765 A2**
(43) Date of publication of application: **10.12.2025**
(21) Application number: 25191909.8
(22) Date of filing: 12.03.2015
(51) Int. Cl.: A61K 48/00

(54) **DYSTROPHIN GENE EXON DELETION USING ENGINEERED NUCLEASES**

(30) Priority: 12.03.2014 US 201461951648 P
(62) Divisional of application: 20189872.3
(71) Applicant: Precision Biosciences, Inc., Durham, NC 27701 (US)
(72) Inventor: JANTZ, Derek, Durham, 27701 (US); SMITH, James Jefferson, Morrisville, 27560 (US); NICHOLSON, Michael G., Chapel Hill, 27517 (US)
(74) Representative: Grund, Martin

(57) **Abstract**

The invention relates to the field of molecular biology and recombinant nucleic acid technology. In particular, the invention relates to a method of treating a patient with Duchenne Muscular Dystrophy comprising the removal of at least one exon from the dystrophin gene using engineered nucleases.

## Description

### RELATED APPLICATIONS

This application claims benefit of priority to U.S. Provisional Patent Applicatoin No. 61/951,648, filed March 12, 2014, the entire disclosure of which is hereby incorporated by reference.

### FIELD OF THE INVENTION

The invention relates to the field of molecular biology and recombinant nucleic acid technology. In particular, the invention relates to a method of treating a patient with Duchenne Muscular Dystrophy comprising the removal of at least one exon from the dystrophin gene using engineered nucleases.

### BACKGROUND OF THE INVENTION

Duchenne Muscular Dystrophy is a rare, X-linked muscle degenerative disorder that affects about 1 in every 3500 boys worldwide. The disease is caused by mutations in the dystrophin *(DMD)* gene, which is the largest known gene. *DMD* spans 2.2 Mb of the X chromosome and encodes predominantly a 14-kb transcript derived from 79 exons. The full-length dystrophin protein, as expressed in skeletal muscle, smooth muscle, and cardiomyocytes, is 3685 amino acids and has a molecular weight of 427 kD. The severe Duchenne phenotype is generally associated with the loss of full length dystrophin protein from skeletal and cardiac muscle, which leads to debilitating muscle degeneration and, ultimately, heart failure. A large number of different *DMD* mutations have been described, many of them resulting in either the severe Duchenne Muscular Dystrophy or the milder Becker Muscular Dystrophy. The Leiden University Medical Center maintains a database of mutations in the *DMD* gene (http://www.dmd.nl).

There are several therapeutic strategies being pursued for the treatment of Duchenne Muscular Dystrophy. First, "gene replacement" strategies are an active area of research (Oshima, et al. (2009) J of the Am. Soc. of Gene Ther. 17:73-80; Liu, et al. (2005) Mol. Ther. 11:245-256; Lai, et al. (2006) Hum Gene Ther. 17:1036-1042; Odom et al. (2008) Mol. Ther. 16:1539-1545). This approach involves delivering a functional copy of the *DMD* gene to patients using a viral delivery vector, typically adeno-associated virus (AAV). The large size of the *DMD* gene makes it incompatible with the limited carrying capacity of common viral vectors, however. This necessitates the use of a "micro-dystrophin" gene in which most of the repetitive central portion of the gene is removed to leave only the minimal functional protein. It is not clear, however, that expression of "micro-dystrophin" is sufficient for clinical benefit. In addition, this approach suffers from the possibility of random gene integration into the patient genome, which could lead to insertional mutagenesis, and the potential for immune reactions against the delivery vector.

A second approach to treating Duchenne Muscular Dystrophy involves the transplantation of healthy muscle precursor cells into patient muscle fibres (Peault et al. (2007) Mol. Ther. 15:867-877; Skuk, et al. (2007) Neuromuscul. Disord. 17:38-46). This approach suffers from inefficient migration of the transplanted myoblasts and the potential for immune rejection by the patient.

A third approach involves suppression of nonsense mutations using PTC124 (Welch, et al. (2007) Nature 447:87-91). This would require lifelong dosing of the drug, however, and the approach is yet to show any significant clinical benefit.

A fourth, and more promising, potential treatment for Duchenne Muscular Dystrophy is called "Exon Skipping" (Williams, et al. (2008) BMC Biotechnol. 8:35*;* Jearawiriyapaisam et al. (2008) Mol Ther. 16:1624-1629; Yokota, et al. (2007) Acta Myol. 26:179-184; van Deutekom et al. (2001) Hum. Mol. Gen. 10: 1547-1554; Benedetti et al. (2013) FEBS J. 280:4263-80; Rodino-Klapac (2013) Curr Neurol Neurosci Rep. 13:332*;* Verhaart and Aartsma-Rus (2012) Curr Opin Neurol. 25:588-96). In general, the N- and C-terminal portions of the dystrophin gene are essential for its role as a "scaffold" protein that maintains membrane integrity in muscle fibres whereas the central "rod domain", which comprises 24 spectrin-like repeats, is at least partially dispensible. Indeed, the severe Duchenne phenotype is typically associated with mutations in the dystrophin gene that introduce frameshifts and/or premature termination codons, resulting in a truncated form of the dystrophin protein lacking the essential C-terminal domain. Mutations in the central rod domain, including large deletions of whole exons, typically result in the much milder Becker phenotype if they maintain the reading frame such that the C-terminal domain of the protein is intact.

Duchenne Muscular Dystrophy is most frequently caused by the deletion of one or more whole exon(s), resulting in reading frame shift. For example, Exon 45 is frequently deleted in Duchenne patients. Because Exon 45 is 176 bp long, which is not divisible by three, deleting the exon shifts Exons 46-79 into the wrong reading frame. The same can be said of Exon 44, which is 148 bp in length. However, if Exons 44 *and* 45 are deleted, the total size of the deletion is 324 bp, which is divisible by three. Thus, the deletion of *both* exons does not result in a reading frame shift. Because these exons encode a portion of the non-essential rod domain of the dystrophin protein, deleting them from the protein is expected to result in a mild Becker-like phenotype. Thus, a patient with the Duchenne phenotype due to the deletion of one or more exon(s) can, potentially, be treated by eliminating one or more adjacent exons to restore the reading frame. This is the principle behind "Exon Skipping," in which modified oligonucleotides are used to block splice acceptor sites in dystrophin pre-mRNA so that one or more specific exons are absent from the processed transcript. The approach has been used to restore dystrophin gene expression in the *mdx* mouse model by skipping Exon 23, which harbored a disease-inducing nonsense mutation (Mann, et al. (2001) Proc. Nat. Acad. Sci. USA 98:42-47). Oligonucleotide analogs which induce skipping of Exon 51 have also shown promise in early human clinical trials (Benedetti et al. (2013) FEBS J. 280:4263-80). The major limitations with this approach are: (1) the exon-skipping process is inefficient, resulting in relatively low levels of functional dystrophin expression; and (2) the exon-skipping oligonucleotide has a relatively short half-life so the affect is transient, necessitating repeated and life-long dosing. Thus, while Exon-Skipping approaches have shown some promise in clinical trials, the improvements in disease progression have been minimal and variable.

The present invention improves upon current Exon-Skipping approaches by correcting gene expression at the level of the genomic DNA rather than pre-mRNA. The invention is a permanent treatment for Duchenne Muscular Dystrophy that involves the excision of specific exons from the *DMD* coding sequence using a pair of engineered, site-specific endonucleases. By targeting a pair of such endonucleases to sites in the intronic regions flanking an exon, it is possible to permanently remove the intervening fragment containing the exon from the genome. The resulting cell, and its progeny, will express mutant dystrophin in which a portion of the non-essential spectrin repeat domain is removed but the essential N- and C-terminal domains are intact.

Methods for producing engineered, site-specific endonucleases are known in the art. For example, zinc-finger nucleases (ZFNs) can be engineered to recognize and cut pre-determined sites in a genome. ZFNs are chimeric proteins comprising a zinc finger DNA-binding domain fused to the nuclease domain of the FokI restriction enzyme. The zinc finger domain can be redesigned through rational or experimental means to produce a protein which binds to a pre-determined DNA sequence ~18 basepairs in length. By fusing this engineered protein domain to the FokI nuclease, it is possible to target DNA breaks with genome-level specificity. ZFNs have been used extensively to target gene addition, removal, and substitution in a wide range of eukaryotic organisms (reviewed in S. Durai et al., Nucleic Acids Res 33, 5978 (2005)). Likewise, TAL-effector nucleases (TALENs) can be generated to cleave specific sites in genomic DNA. Like a ZFN, a TALEN comprises an engineered, site-specific DNA-binding domain fused to the FokI nuclease domain (reviewed in Mak, et al. (2013) Curr Opin Struct Biol. 23:93-9). In this case, however, the DNA binding domain comprises a tandem array of TAL-effector domains, each of which specifically recognizes a single DNA basepair. A limitation that ZFNs and TALENs have for the practice of the current invention is that they are heterodimeric, so that the production of a single functional nuclease in a cell requires co-expression of two protein monomers. Because the current invention requires *two* nucleases, one to cut on either side of the exon of interest, this would necessitate co-expressing *four* ZFN or TALEN monomers in the same cell. This presents significant challenges in gene delivery because traditional gene delivery vectors have limited carrying capacity. It also introduces the possibility of "mis-dimerization" in which the monomers associate inappropriately to make unintended dimeric endonuclease species that might recognize and cut off-target locations in the genome. This can, potentially, be minimized by generating orthogonal obligate heterodimers in which the FokI nuclease domains of the four monomers are differentially engineered to dimerize preferentially with the intended partner monomer.

Compact TALENs are an alternative endonuclease architecture that avoids the need for dimerization (Beurdeley, et al. (2013) Nat Commun. 4:1762 ). A Compact TALEN comprises an engineered, site-specific TAL-effector DNA-binding domain fused to the nuclease domain from the I-TevI homing endonuclease. Unlike FokI, I-TevI does not need to dimerize to produce a double-strand DNA break so a Compact TALEN is functional as a monomer. Thus, it is possible to co-express two Compact TALENs in the same cell to practice the present invention.

Engineered endonucleases based on the CRISPR/Cas9 system are also know in the art (Ran, et al. (2013) Nat Protoc. 8:2281-2308; Mali et al. (2013) Nat Methods. 10:957-63). A CRISPR endonuclease comprises two components: (1) a caspase effector nuclease, typically microbial Cas9; and (2) a short "guide RNA" comprising a ~20 nucleotide targeting sequence that directs the nuclease to a location of interest in the genome. By expressing multiple guide RNAs in the same cell, each having a different targeting sequence, it is possible to target DNA breaks simultaneously to multiple sites in in the genome. Thus, CRISPR/Cas9 nucleases are suitable for the present invention. The primary drawback of the CRISPR/Cas9 system is its reported high frequency of off-target DNA breaks, which could limit the utility of the system for treating human patients (Fu, et al. (2013) Nat Biotechnol. 31:822-6).

In the preferred embodiment of the invention, the DNA break-inducing agent is an engineered homing endonuclease (also called a "meganuclease"). Homing endonucleases are a group of naturally-occurring nucleases which recognize 15-40 base-pair cleavage sites commonly found in the genomes of plants and fungi. They are frequently associated with parasitic DNA elements, such as group 1 self-splicing introns and inteins. They naturally promote homologous recombination or gene insertion at specific locations in the host genome by producing a double-stranded break in the chromosome, which recruits the cellular DNA-repair machinery (Stoddard (2006), Q. Rev. Biophys. 38: 49-95). Homing endonucleases are commonly grouped into four families: the LAGLIDADG family, the GIY-YIG family, the His-Cys box family and the HNH family. These families are characterized by structural motifs, which affect catalytic activity and recognition sequence. For instance, members of the LAGLIDADG family are characterized by having either one or two copies of the conserved LAGLIDADG motif (see Chevalier et al. (2001), Nucleic Acids Res. 29(18): 3757-3774). The LAGLIDADG homing endonucleases with a single copy of the LAGLIDADG motif form homodimers, whereas members with two copies of the LAGLIDADG motif are found as monomers.

I-CreI (SEQ ID NO: 1) is a member of the LAGLIDADG family of homing endonucleases which recognizes and cuts a 22 basepair recognition sequence in the chloroplast chromosome of the algae *Chlamydomonas reinhardtii.* Genetic selection techniques have been used to modify the wild-type I-CreI cleavage site preference (Sussman et al. (2004), J. Mol. Biol. 342: 31-41; Chames et al. (2005), Nucleic Acids Res. 33: e178; Seligman et al. (2002), Nucleic Acids Res. 30: 3870-9, Arnould et al. (2006), J. Mol. Biol. 355: 443-58). More recently, a method of rationally-designing mono-LAGLIDADG homing endonucleases was described which is capable of comprehensively redesigning I-CreI and other homing endonucleases to target widely-divergent DNA sites, including sites in mammalian, yeast, plant, bacterial, and viral genomes (WO 2007/047859).

As first described in WO 2009/059195, I-CreI and its engineered derivatives are normally dimeric but can be fused into a single polypeptide using a short peptide linker that joins the C-terminus of a first subunit to the N-terminus of a second subunit (Li, et al. (2009) Nucleic Acids Res. 37:1650-62; Grizot, et al. (2009) Nucleic Acids Res. 37:5405-19.) Thus, a functional "single-chain" meganuclease can be expressed from a single transcript. By delivering genes encoding two different single-chain meganucleases to the same cell, it is possible to simultaneously cut two different sites. This, coupled with the extremely low frequency of off-target cutting observed with engineered meganucleases makes them the preferred endonuclease for the present invention.

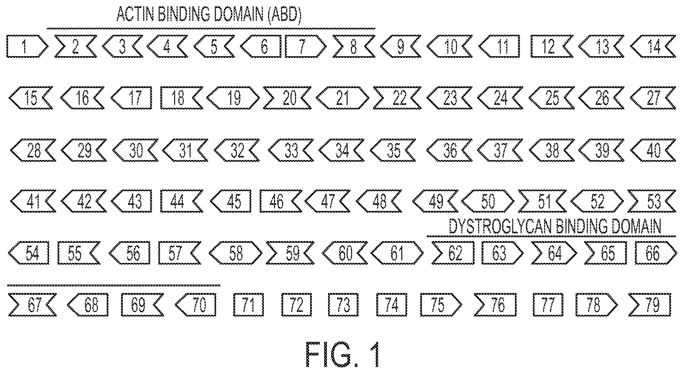

The use of engineered meganucleases for treatment of Duchenne Muscular Dystrophy was previously disclosed in WO 2011/141820 (the '820 application). In this patent application, the authors discuss the possibility of using engineered meganucleases to correct defects in the *DMD* gene via three different mechanisms (see WO 2011/141820 Figure 1). First, the authors contemplate the use of an engineered meganuclease to insert a transgenic copy of *DMD* or micro-DMD into a "safe harbor" locus, such as AAVS1, where it will be expressed constitutively without affecting endogenous gene expression. Second, the authors propose that a meganuclease might be made to cleave the genome at a site near a deleterious mutation in *DMD* and that this DNA break would stimulate homologous recombination between the mutant *DMD* gene in the genome and a healthy copy of the gene provided *in trans* such that the mutation in the genome would be corrected. Third, the authors of the '820 application propose that an engineered meganuclease can be made to insert foreign DNA into an intron in the *DMD* gene and that such a meganuclease could be used insert the essential C-terminal domain of dystrophin into an early intron upstream of a mutation causing disease. Significantly, in contemplating the myriad uses of meganucleases for manipulating the *DMD* gene, the authors of the '820 application do not contemplate the use of two meganucleases simultaneously in the same cells, nor do they propose the *removal* of any DNA sequence as in the present invention.

Finally, Ousterout *et al.* demonstrated that a DNA break can be targeted to the DMD coding sequence using a TALEN and that the break is frequently repaired via the mutagenic non-homologous end-joining pathway, resulting in the introduction of small insertions and/or deletions ("indels") that can change the reading frame of the gene (Ousterout et al. (2013) Mol Ther. 21:1718-26). They demonstrated the possibility of restoring *DMD* gene expression in a portion of mutant cells by delivering a DNA break to the exon immediately following the mutation and relying on mutagenic DNA repair to restore the reading frame in some percentage of cells. Unlike the present invention, this approach involved a single nuclease and was targeted to the coding sequence of the gene.

### SUMMARY OF THE INVENTION

The present invention is a method of treating Duchenne Muscular Dystrophy comprising delivering a pair of engineered nucleases, or genes encoding engineered nucleases, to the muscle cells of a patient such that the two nucleases excise one or more exons of the *DMD* gene to restore the normal reading frame. Cells so treated will express a shortened form of the dystrophin protein in which a portion of the central spectrin repeat domain is absent but the N- and C-terminal domains are intact. This will, in many cases, reduce the severity of the disease to the mild Becker phenotype.

Thus, in one embodiment, the invention provides a general method for treating Duchenne Muscular Dystrophy using a pair of nucleases. In another embodiment, the invention provides engineered meganucleases suitable for practicing the method. In a third embodiment, the invention provides engineered Compact TALENs suitable for practicing the method. In a fourth embodiment, the invention provides CRISPRs for practicing the method. In a fifth embodiment, the invention provides vectors and techniques for delivering engineered nucleases to patient cells.

### BRIEF DESCRIPTION OF THE FIGURES

Figure 1. Structure of the *DMD* gene. 79 exons are drawn to indicate reading frame. The essential Actin-binding and Dystroglycan-binding domains, which span approximately Exons 2-8 and 62-70, respectively, are indicated.

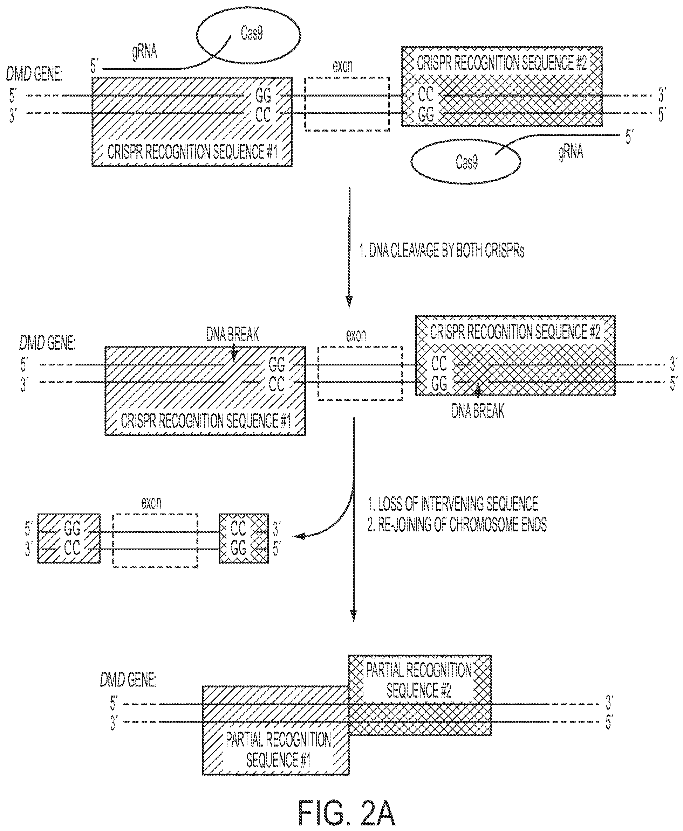

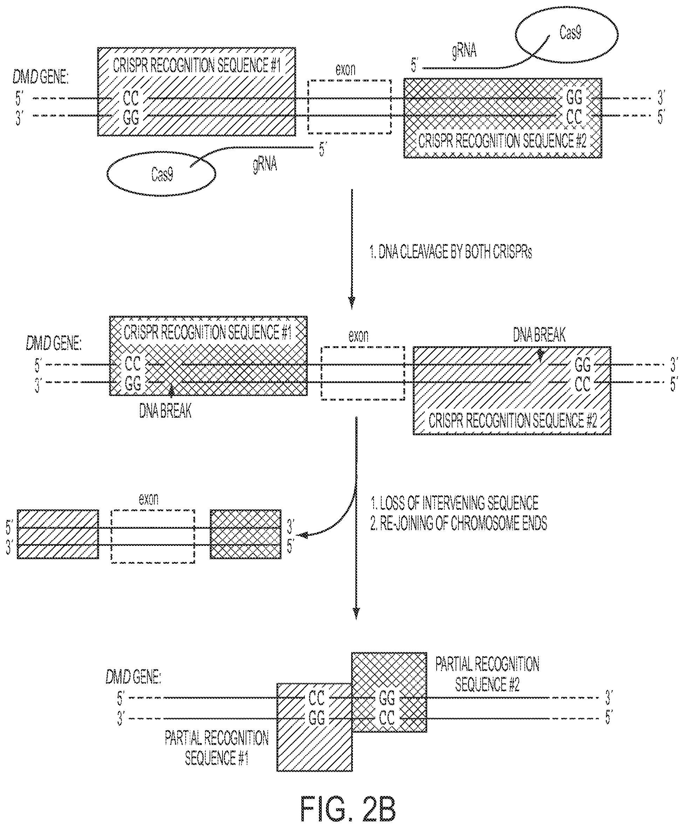

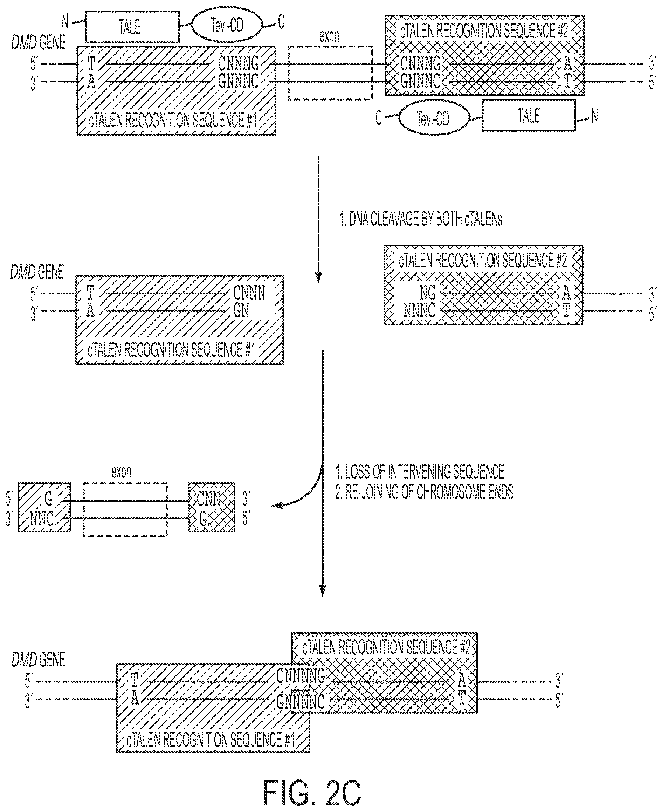

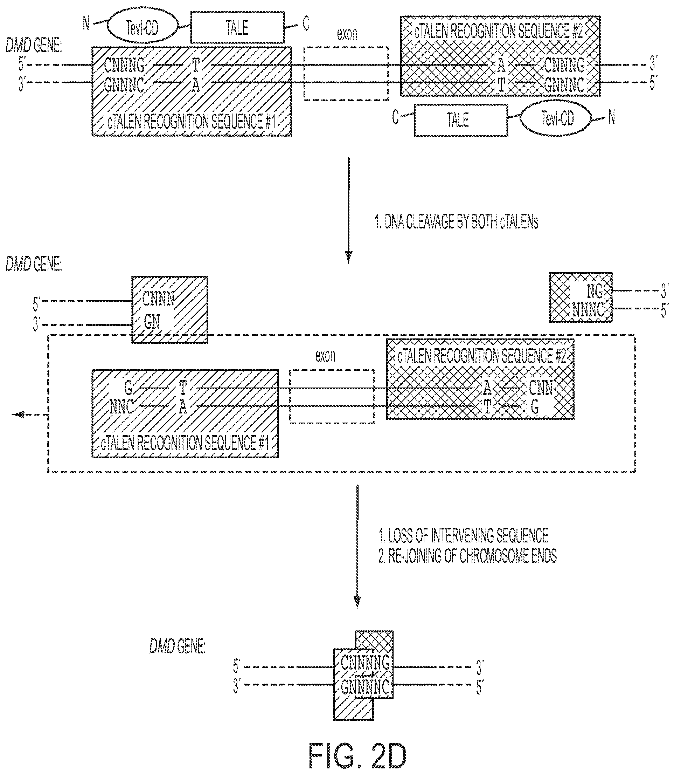

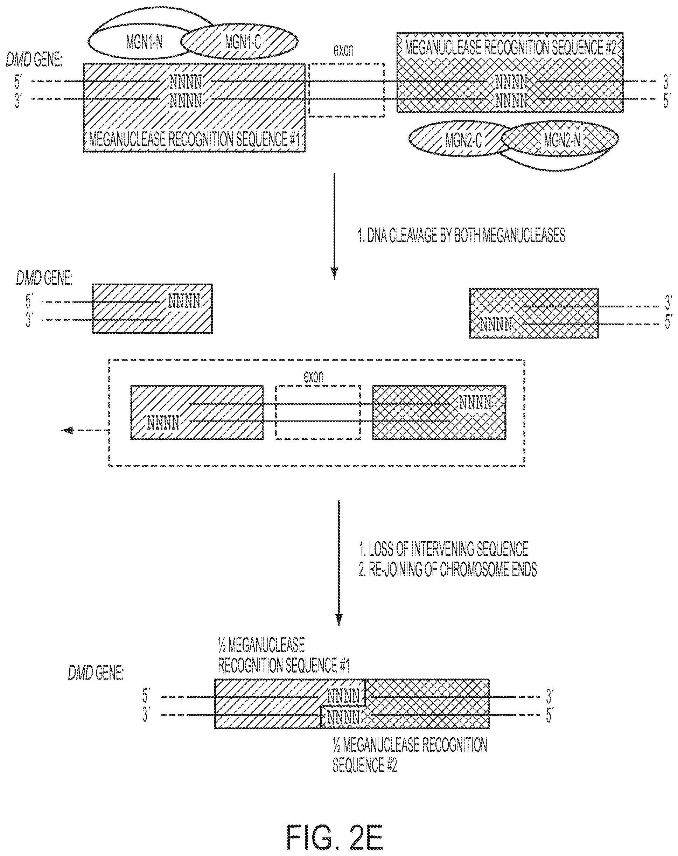

Figure 2. Strategies for deleting exons from the *DMD* gene using different types of nucleases. 2A) Strategy for deleting an exon using a pair of CRISPRs. A pair of "guide RNAs" ("gRNAs") are used which are complementary to a pair of recognition sites flanking the exon of interest. As drawn in this figure, the gRNAs can be complementary to recognition sequences that are distal to the conserved "GG" motif and the site of Cas9 DNA cleavage. In this orientation, the CRISPR recognition sequences are largely conserved following DNA cleavage, excision of the intervening fragment of genomic DNA, and re-joining of the chromosome ends. 2B) An alternative scheme for deleting an exon using a pair of CRISPRs in which the gRNAs are complementary to recognition sequences that are proximal to the exon. In this orientation, the CRISPR recognition sequences are largely deleted following DNA cleavage, excision of the intervening fragment of genomic DNA, and re-joining of the chromosome ends. It is contemplated in the invention could also comprise a hybrid of the schemes shown in 2A and 2B. 2C) Strategy for deleting an exon using a pair of compact TALENs (cTALENs). A pair of TAL effector DNA-binding domains ("TALEs") are used which bind to a pair of recognition sites flanking the exon of interest. As drawn in this figure, the TALEs can bind to recognition sequences that are distal to the conserved "CNNNG" motif that is recognized and cut by the I-TevI cleavage domain ("TevI-CD"). In this orientation, the cTALEN recognition sequences are largely conserved following DNA cleavage, excision of the intervening fragment of genomic DNA, and re-joining of the chromosome ends. Also, the cTALENs in this figure are shown with the TALE and TevI-CD domains in an N- to C- orientation. It is also possible to generate cTALENs with these two domains in a C- to N- orientation. 2D) An alternative scheme for deleting an exon using a pair of cTALENS in which the TALE domains bind to recognition sequences that are proximal to the exon. In this orientation, the cTALEN recognition sequences are largely deleted following DNA cleavage, excision of the intervening fragment of genomic DNA, and re-joining of the chromosome ends. Also, the cTALENs in this figure are drawn with the TALE and TevI-CD domains in a C- to N- orientation. It is contemplated in the invention could also comprise a hybrid of the schemes shown in 2C and 2D. 2E) Strategy for deleting an exon from the *DMD* gene using a pair of single-chain meganucleases. The meganucleases are drawn as two-domain proteins (MGN-N: the N-terminal domain; and MGN-C: the C-terminal domain) joined by a linker. In the figure, the C-terminal domain is drawn as binding to the half of the recognition sequence that is closest to the exon. In some embodiments, however, the N-terminal domain can bind to this half of the recognition sequence. The central four basepairs of the recognition sequence are shown as "NNNN". These four basepairs become single-strand 3' "overhangs" following cleavage by the meganuclease. The subset of preferred four basepair sequences that comprise this region of the sequence are identified in WO/2010/009147. DNA cleavage by the pair of meganucleases generates a pair of four basepair 3' overhangs at the chromosome ends. If these overhangs are complementary, they can anneal to one another and be directly re-ligated, resulting in the four basepair sequence being retained in the chromosome following exon excision. Because meganucleases cleave near the middle of the recognition sequence, half of each recognition sequence will frequently be retained in the chromosome following excision of the exon. The other half of each recognition sequence will removed from the genome with the exon.

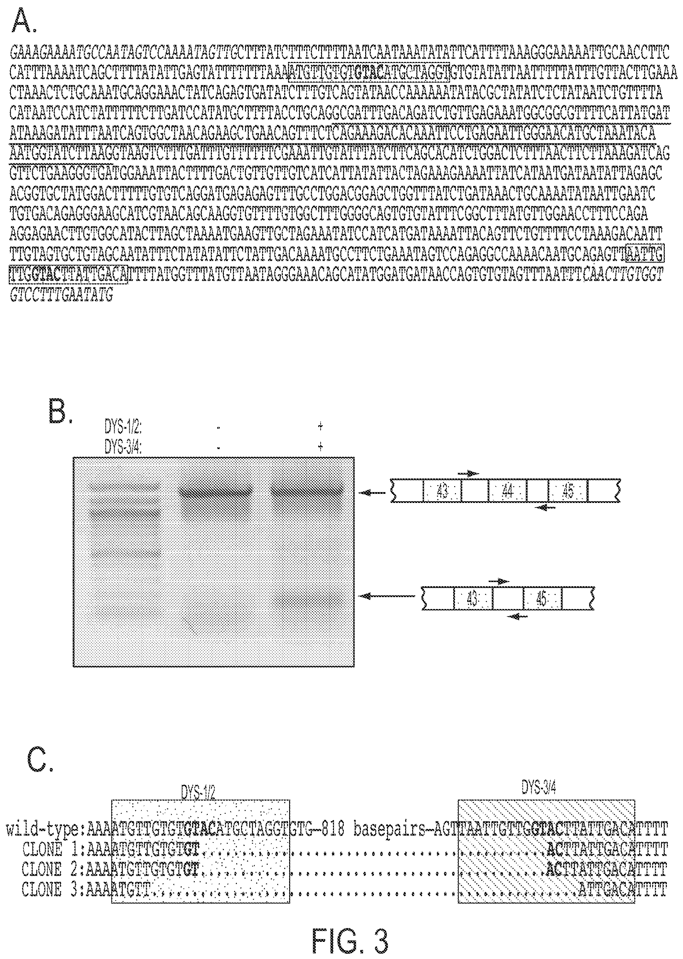

Figure 3. Excision of *DMD* Exon 44 using the DYS-1/2 and DYS-3/4 meganucleases. 3A) Sequence of *DMD* Exon 44 and flanking regions. The Exon sequence is underlined. Recognition sites for the DYS-1/2 and DYS-3/4 meganucleases are shaded in gray with the central four basepairs (which become the 3' overhang following cleavage by the meganuclease) in bold. Annealing sites for a pair of PCR primers used for analysis are italicized. 3B) Agarose gel electrophoresis analysis of HEK-293 cells co-expressing DYS-1/2 and DYS-3/4. Genomic DNA was isolated from the cells and evaluated by PCR using the primers indicated in (3A). PCR products were resolved on an agarose gel and it was found that HEK-293 cells co-expressing the two meganucleases yielded a pair of PCR bands whereas wild-type HEK-293 cells yielded only the larger band. 3C) sequences from three plasmids harboring the smaller PCR product from (3B). The three sequences are shown aligned to the wild-type human sequence. The locations of the DYS-1/2 and DYS-3/4 recognition sequences are shaded in gray with the central four basepairs in bold.

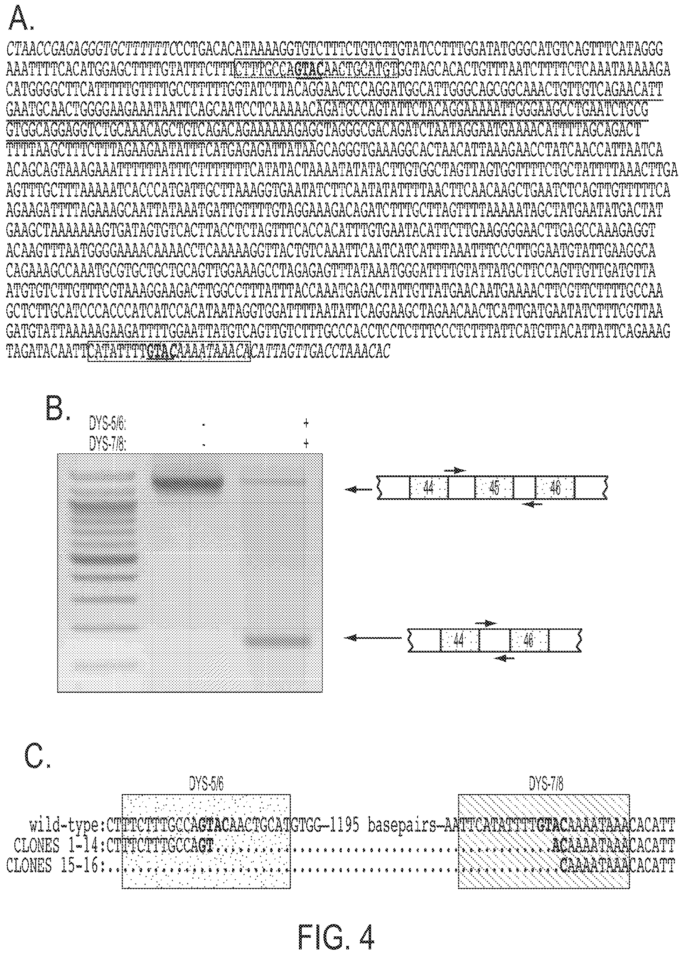

Figure 4. Excision of *DMD* Exon 45 using the DYS-5/6 and DYS-7/8 meganucleases. 4A) Sequence of *DMD* Exon 45 and flanking regions. The Exon sequence is underlined. Recognition sites for the DYS-5/6 and DYS-7/8 meganucleases are shaded in gray with the central four basepairs (which become the 3' overhang following cleavage by the meganuclease) in bold. Annealing sites for a pair of PCR primers used for analysis are italicized. 4B) Agarose gel electrophoresis analysis of HEK-293 cells co-expressing DYS-5/6 and DYS-7/8. Genomic DNA was isolated from the cells and evaluated by PCR using the primers indicated in (4A). PCR products were resolved on an agarose gel and it was found that HEK-293 cells co-expressing the two meganucleases yielded a pair of PCR bands whereas wild-type HEK-293 cells yielded only the larger band. 4C) sequences from 16 plasmids harboring the smaller PCR product from (4B). The sequences are shown aligned to the wild-type human sequence. The locations of the DYS-5/6 and DYS-7/8 recognition sequences are shaded in gray with the central four basepairs in bold.

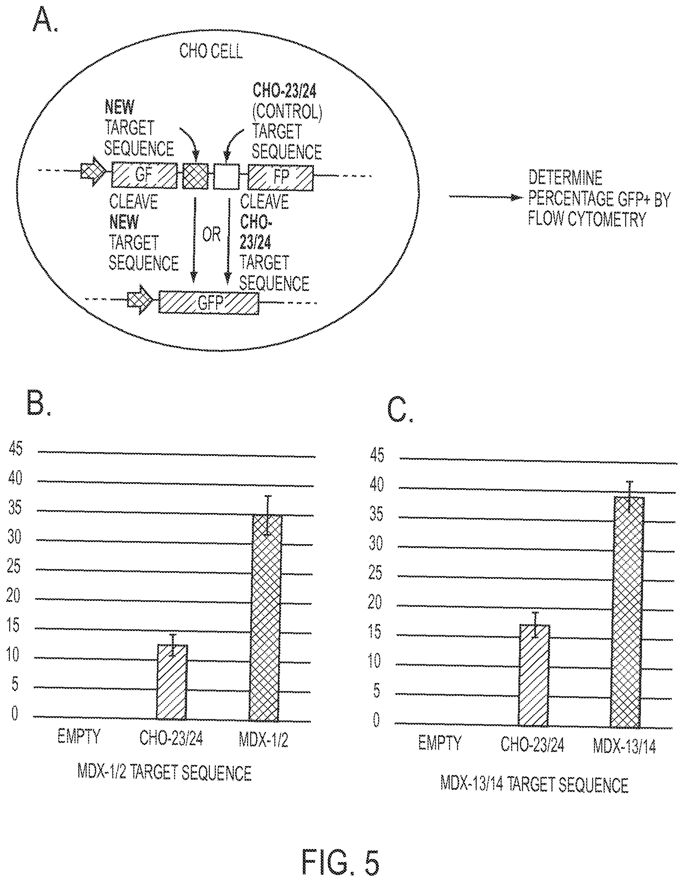

Figure 5. Evaluation of the MDX-1/2 and MDX-13/14 meganucleases in a reporter assay in CHO cells. A) Schematic of the assay. For each of the two meganucleases, we produced a CHO cell line in which a reporter cassette was integrated stably into the genome of the cell. The reporter cassette comprised, in 5' to 3' order: an SV40 Early Promoter; the 5' 2/3 of the GFP gene; the recognition site for either MDX-1/2 (SEQ ID NO: 149) or the recognition site for MDX-13/14 (SEQ ID NO: 150); the recognition site for the CHO-23/24 meganuclease (WO/2012/167192); and the 3' 2/3 of the GFP gene. Cells stably transfected with this cassette did not express GFP in the absence of a DNA break-inducing agent. When a DNA break was induced at either of the meganuclease recognition sites, however, the duplicated regions of the GFP gene recombined with one another to produce a functional GFP gene. The percentage of GFP-expressing cells could then be determined by flow cytometry as an indirect measure of the frequency of genome cleavage by the meganucleases. B,C) The two CHO reporter lines were transfected with mRNA encoding the MDX-1/2 (A), MDX-13/14 (B), or CHO-23/34 (A and B) meganucleases. 1.5e6 CHO cells were transfected with 1e6 copies of mRNA per cell using a Lonza Nucleofector 2 and program U-024 according to the manufacturer's instructions. 48 hours post-transfection, the cells were evaluated by flow cytometry to determine the percentage of GFP-positive cells compared to an untransfected (Empty) negative control. The assay was performed in triplicate and standard deviations are shown. The MDX-1/2 and MDX-13/14 meganucleases were found to produce GFP+ cells in their respective cell lines at frequencies significantly exceeding both the negative (Empty) control and the CHO-23/24 postive control, indicating that the nucleases are able to efficiently recognize and cut their intended target sequences in a cell.

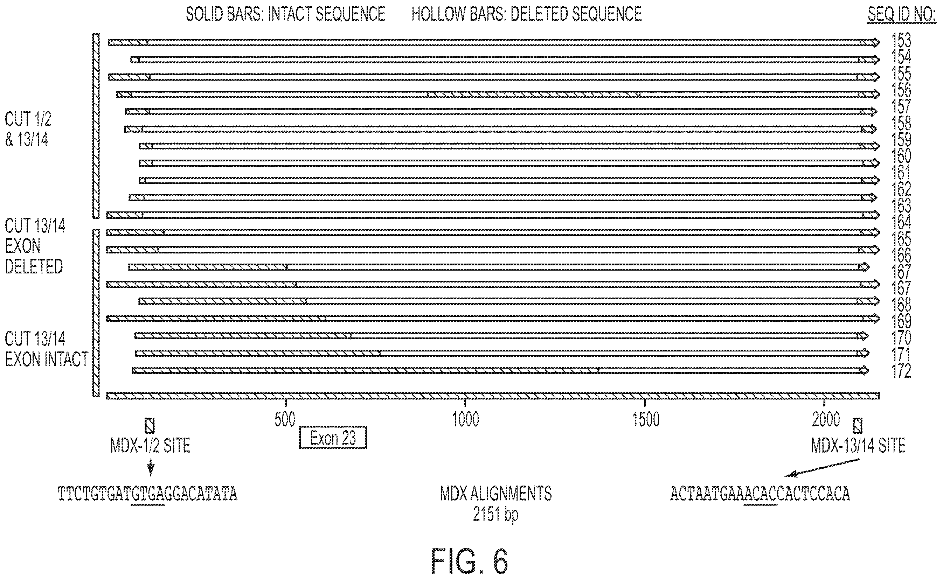

Figure 6. Sequence alignments from 20 C2C12 mouse myoblast clones in which a portion of the *DMD* gene was deleted by co-transfection with the MDX-1/2 and MDX-13/14 meganucleases. The location of *DMD* Exon 23 is shown as are the locations and sequences of the MDX-1/2 and MDX-13/14 target sites. Each of the 20 sequences (SEQ ID NO: 153-172) was aligned to a reference wild-type *DMD* sequence and deletions relative to the reference are shown as hollow bars.

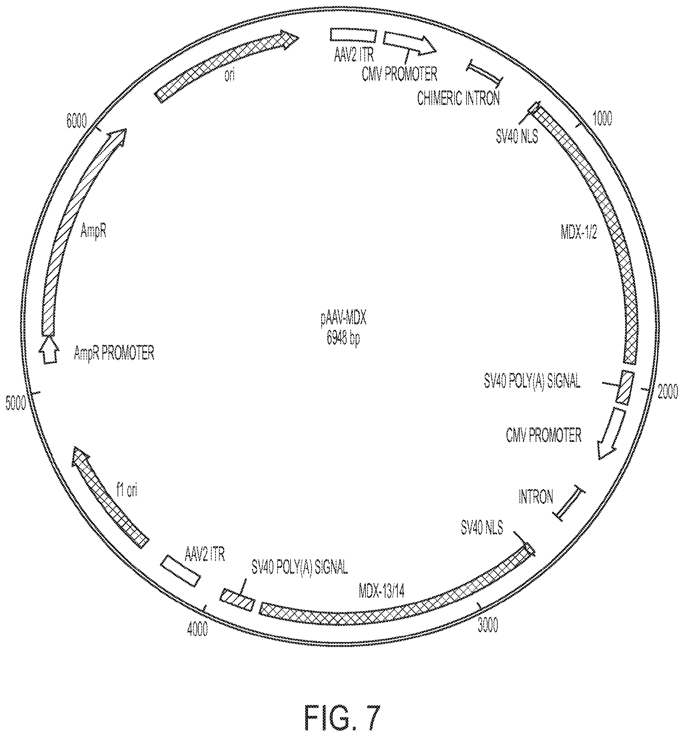

Figure 7. Vector map of the pAAV-MDX plasmid. This "packaging" plasmid was used in conjunction with an Ad helper plasmid to produce AAV virus capable of simultaneously delivering the genes encoding the MDX-1/2 and MDX-13/14 meganucleases.

### DETAILED DESCRIPTION OF THE INVENTION

### 1.1 References and Definitions

The patent and scientific literature referred to herein establishes knowledge that is available to those of skill in the art. The issued U.S. patents, allowed applications, published foreign applications, and references, including GenBank database sequences, that are cited herein are hereby incorporated by reference to the same extent as if each was specifically and individually indicated to be incorporated by reference.

As used herein, the term "meganuclease" refers to an endonuclease that is derived from I-CreI. The term meganuclease, as used herein, refers to an engineered variant of I-CreI that has been modified relative to natural I-CreI with respect to, for example, DNA-binding specificity, DNA cleavage activity, DNA-binding affinity, or dimerization properties. Methods for producing such modified variants of I-CreI are known in the art *(e.g.* WO 2007/047859). A meganuclease may bind to double-stranded DNA as a homodimer, as is the case for wild-type I-CreI, or it may bind to DNA as a heterodimer. A meganuclease may also be a "single-chain meganuclease" in which a pair of DNA-binding domains derived from I-CreI are joined into a single polypeptide using a peptide linker.

As used herein, the term "single-chain meganuclease" refers to a polypeptide comprising a pair of meganuclease subunits joined by a linker. A single-chain meganuclease has the organization: N-terminal subunit - Linker - C-terminal subunit. The two meganuclease subunits, each of which is derived from I-CreI, will generally be non-identical in amino acid sequence and will recognize non-identical DNA sequences. Thus, single-chain meganucleases typically cleave pseudo-palindromic or non-palindromic recognition sequences. A single chain meganuclease may be referred to as a "single-chain heterodimer" or "single-chain heterodimeric meganuclease" although it is not, in fact, dimeric. For clarity, unless otherwise specified, the term "meganuclease" can refer to a dimeric or single-chain meganuclease.

As used herein, the term "Compact TALEN" refers to an endonuclease comprising a DNA-binding domain with 16-22 TAL domain repeats fused in any orientation to any portion of the I-TevI homing endonuclease.

As used herein, the term "CRISPR" refers to a caspase-based endonuclease comprising a caspase, such as Cas9, and a guide RNA that directs DNA cleavage of the caspase by hybridizing to a recognition site in the genomic DNA.

As used herein, with respect to a protein, the term "recombinant" means having an altered amino acid sequence as a result of the application of genetic engineering techniques to nucleic acids which encode the protein, and cells or organisms which express the protein. With respect to a nucleic acid, the term "recombinant" means having an altered nucleic acid sequence as a result of the application of genetic engineering techniques. Genetic engineering techniques include, but are not limited to, PCR and DNA cloning technologies; transfection, transformation and other gene transfer technologies; homologous recombination; site-directed mutagenesis; and gene fusion. In accordance with this definition, a protein having an amino acid sequence identical to a naturally-occurring protein, but produced by cloning and expression in a heterologous host, is not considered recombinant.

As used herein, the term "wild-type" refers to any naturally-occurring form of a meganuclease. The term "wild-type" is not intended to mean the most common allelic variant of the enzyme in nature but, rather, any allelic variant found in nature. Wild-type homing endonucleases are distinguished from recombinant or non-naturally-occurring meganucleases.

As used herein, the term "recognition sequence" refers to a DNA sequence that is bound and cleaved by an endonuclease. In the case of a meganuclease, a recognition sequence comprises a pair of inverted, 9 basepair "half sites" which are separated by four basepairs. In the case of a single-chain meganuclease, the N-terminal domain of the protein contacts a first half-site and the C-terminal domain of the protein contacts a second half-site. Cleavage by a meganuclease produces four basepair 3' "overhangs". "Overhangs", or "sticky ends" are short, single-stranded DNA segments that can be produced by endonuclease cleavage of a double-stranded DNA sequence. In the case of meganucleases and single-chain meganucleases derived from I-CreI, the overhang comprises bases 10-13 of the 22 basepair recognition sequence. In the case of a Compact TALEN, the recognition sequence comprises a first CNNNGN sequence that is recognized by the I-TevI domain, followed by a non-specific spacer 4-16 basepairs in length, followed by a second sequence 16-22 bp in length that is recognized by the TAL-effector domain (this sequence typically has a 5' T base). Cleavage by a Compact TALEN produces two basepair 3' overhangs. In the case of a CRISPR, the recognition sequence is the sequence, typically 16-24 basepairs, to which the guide RNA binds to direct Cas9 cleavage. Cleavage by a CRISPR produced blunt ends.

As used herein, the term "target site" or "target sequence" refers to a region of the chromosomal DNA of a cell comprising a recognition sequence for a meganuclease.

As used herein, the term "homologous recombination" or "HR" refers to the natural, cellular process in which a double-stranded DNA-break is repaired using a homologous DNA sequence as the repair template (see, *e.g.* Cahill et al. (2006), Front. Biosci. 11:1958-1976). The homologous DNA sequence may be an endogenous chromosomal sequence or an exogenous nucleic acid that was delivered to the cell.

As used herein, the term "non-homologous end-joining" or "NHEJ" refers to the natural, cellular process in which a double-stranded DNA-break is repaired by the direct joining of two non-homologous DNA segments (see, *e.g.* Cahill et al. (2006), Front. Biosci. 11:1958-1976). DNA repair by non-homologous end-joining is error-prone and frequently results in the untemplated addition or deletion of DNA sequences at the site of repair.

As used herein, the term "re-ligation" refers to a process in which two DNA ends produced by a pair of double-strand DNA breaks are covalently attached to one another with the loss of the intervening DNA sequence but without the gain or loss of any additional DNA sequence. In the case of a pair of DNA breaks that are produced with single-strand overhangs, re-ligation can proceed via annealing of complementary overhangs followed by covalent attachment of 5' and 3' ends by a DNA ligase. Re-ligation is distinguished from NHEJ in that it it does not result in the untemplated addition or removal of DNA from the site of repair.

As used herein, unless specifically indicated otherwise, the word "or" is used in the inclusive sense of "and/or" and not the exclusive sense of "either/or."

### 2.1 Principle of Exon Deletion

The present invention is based, in part, on the hypothesis that certain deletions in the *DMD* gene that give rise to the Duchenne phenotype can be compensated for by deleting (an) additional exon(s) immediately up- or downstream of the mutation. The DMD-Leiden Database indicates that most of the mutations that cause Duchenne Muscular Dystrophy are deletions of one or more whole exons that cause a shift in reading frame. In many cases, the reading frame can be restored by eliminating the exon immediately before or after the mutation. As shown in Table 1, 29 different Duchenne-causing mutations, representing ~65% of patients, can be compensated for by deleting a single exon adjacent to the mutation. For example, a patient with disease due to the deletion of *DMD* Exon 45, which occurs in approximately 7% of patients, can be treated with a therapeutic that deletes Exon 46. Notably, a therapeutic capable of deleting Exon 51 or Exon 45 could be used to treat 15% and 13% of patients, respectively.

**Table 1**

| **Exon(s) deleted in patient** | **Additional Exon to delete** | **Frequency in DMD-Leiden Database (%)** |
|---|---|---|
| 44, 44-47 | 43 | 5 |
| 35-43, 45, 45-54 | 44 | 8 |
| 18-44, 44, 46-47, 46-48, 46-49, 46-51, 46-53 | 45 | 13 |
| 45 | 46 | 7 |
| 51, 51-55 | 50 | 5 |
| 50, 45-50, 48-50, 49-50, 52, 52-63 | 51 | 15 |
| 51, 53, 53-55 | 52 | 3 |
| 45-52, 48-52, 49-52, 50-52, 52 | 53 | 9 |

### 2.2 Nucleases for Deleting Exons

It is known in the art that it is possible to use a site-specific nuclease to make a DNA break in the genome of a living cell and that such a DNA break can result in permanent modification of the genome via mutagenic NHEJ repair or via HR with a transgenic DNA sequence. The present invention, however, involves co-expression of a pair of nucleases in the same cell. Surprisingly, we found that a pair of nucleases targeted to DNA sites in close proximity to one another (less than 10,000 basepairs apart) can excise the intervening DNA fragment from the genome. Also surprisingly, we found that DNA excision using a pair of nucleases frequently proceeds via a mechanism involving the single-stranded DNA overhangs generated by the nucleases. In experiments involving a pair of meganucleases that generate complementary *(i.e.* identical) DNA overhangs, it was found that the overhang sequence was frequently conserved following fragment excision and repair of the resulting chromosome ends (see Examples 1 and 2). The mechanism of DNA repair, in this case, appears to direct re-ligation of the broken ends, which has not been observed in mammalian cells. Such precise deletion and re-ligation was not observed when using a pair of meganucleases that generated non-identical overhangs (see Example 3). Thus, in a preferred embodiment, the pair of nucleases used for *DMD* exon excision are selected to generate complementary overhangs.

To excise an exon efficiently, the pair of nuclease cut sites need to be relatively close together. In general, the closer the two sites are to one another, the more efficient the process will be. Thus, the preferred embodiment of the invention uses a pair of nucleases that cut sequences that are less than 10,000 basepairs or, more preferably, 5,000 basepairs or, still more preferably, less than 2,500 basepairs, or, most preferably, less than 1,500 basepairs apart.

As shown in Figure 2, a variety of different types of nuclease are useful for practicing the invention. Figures 2A and 2B show examples of how the invention can be practiced using a pair of CRISPR nucleases. In this case, the invention can be practiced by delivering three genes to the cell: one gene encoding the Cas9 protein and one gene encoding each of the two guide RNAs. CRISPRs cleave DNA to leave blunt ends which are not generally re-ligated cleanly such that the final product will generally have additiona insertion and/or deletion ("indel") mutations in the sequence. In an alternative embodiment, a "CRISPR Nickase" may be used, as reported in Ran, et al. (2013) Cell. 154:1380-9. To practice this embodiment, it is necessary to express four guide RNAs in the cell, two of which are complementary to the sequence upstream of the exon and two of which are complementary to the sequence downstream of the exon. In this embodiment, the two pairs of guide RNAs hybridize with complementary strands in the target region and each member of the pair produces a single strand DNA nick on one of the strands. The result is a pair of nicks (equivalent to a double-strand break) that can be off-set from one another to yield a single-strand overhang that is advantageous for practicing the invention. Methods for making CRISPRs and CRISPR Nickases that recognize pre-determined DNA sites are known in the art, for example Ran, et al. (2013) Nat Protoc. 8:2281-308.

In alternative embodiments, as diagrammed in Figure 2C and 2D, the nuclease pair can be Compact TALENs. A compact TALEN comprises a TAL-effector DNA-binding domain (TALE) fused at its N- or C-terminus to the cleavage domain from I-TevI, comprising at least residues 1-96 and preferably residues 1-182 of I-TevI. The I-TevI cleavage domain recognizes and cuts DNA sequences of the form 5'-CNbNNtG-3', where "b" represents the site of cleavage of the bottom strand and "t" represents the site of cleavage of the top strand and where"N" is any of the four bases. A Compact TALEN, thus, cleaves to produce two basepair 3' overhangs. In a preferred embodiment, the Compact TALEN pair used for exon excision is selected to have complementary overhangs that can directly re-ligate. Methods for making TALE domains that bind to pre-determined DNA sites are known in the art, for example Reyon et al. (2012) Nat Biotechnol. 30:460-5*.*

In the preferred embodiment, as diagrammed in Figure 2E, the nucleases used to practice the invention are a pair of Single-Chain Meganucleases. A Single-Chain Meganuclease comprises an N-terminal domain and a C-terminal domain joined by a linker peptide. Each of the two domains recognizes half of the Recognition Sequence and the site of DNA cleavage is at the middle of the Recognition Sequence near the interface of the two subunits. DNA strand breaks are offset by four basepairs such that DNA cleavage by a meganuclease generates a pair of four basepair, 3' single-strand overhangs. In a preferred embodiment, single-chain meganucleases are selected which cut Recognition Sequences with complementary overhangs, as in Examples 1 and 2. Example recognition sequences for *DMD* Exons 44, 45, and 51 are listed in Tables 2-7. To excise Exon 44, for example, a first meganuclease can be selected which cuts a Recognition Sequence from Table 2, which lists Recognition Sequences upstream of Exon 44. A second meganuclease can then be selected which cuts a Recognition sequences from Table 3, which lists Recognition Sequences downstream of Exon 44. Co-expression of the two meganucleases in the same cell will thus excise Exon 44. Preferably, meganucleases are selected which cut DNA to leave complementary single strand overhangs. For example, SEQ ID NO: 19, if cut by a meganuclease, leaves the overhang sequence: 5'-GTAC-3'. Likewise, SEQ ID NO: 42 if cut by a meganuclease, leaves the overhang sequence: 5'-GTAC-3'. Thus, co-expressing a first meganuclease which cleaves SEQ ID NO: 19 with a second meganuclease which cleaves SEQ ID NO:42 will excise *DMD* Exon 44 from the genome of a human cell such that complementary overhangs are produced which can be repaired via direct re-ligation.

**Table 2. Example Meganuclease Recognition Sequences Upstream of DMD Exon 44**

| Recognition Sequence | SEQ ID NO: | Overhang |
|---|---|---|
| TTCTCTGTGGTGAGAAAATTTA | 2 | GTGA |
| TTCACTATTTTGAAATATACAG | 3 | TTGA |
| TATTTTGAAATATACAGCACAA | 4 | ATAT |
| TAACTTTGTTCATATTACTATG | 5 | TCAT |
| ACTTTGTTCATATTACTATGCA | 6 | ATAT |
| CATATTACTATGCAATAGAACA | 7 | ATGC |
| CACTAGAACTTATTACTCCTTT | 8 | TTAT |
| TTTCAGTTGATGAACAGGCAGT | 9 | ATGA |
| AGTTTTGGATCAAGAATAATAT | 10 | TCAA |
| AAAAATATTTTGAAAGGGAATA | 11 | TTGA |
| CCAAATAATTTATTACAATGTT | 12 | TTAT |
| ATCTTTCTTTTAATCAATAAAT | 13 | TTAA |
| TTTTAATCAATAAATATATTCA | 14 | ATAA |
| ACCTTCCATTTAAAATCAGCTT | 15 | TTAA |
| TCAGCTTTTATATTGAGTATTT | 16 | ATAT |
| GCTTTTATATTGAGTATTTTTT | 17 | TTGA |
| TAAAATGTTGTGTGTACATGCT | 18 | GTGT |
| ATGTTGTGTGTACATGCTAGGT | 19 | GTAC |
| GCTAGGTGTGTATATTAATTTT | 20 | GTAT |
| ATTTGTTACTTGAAACTAAACT | 21 | TTGA |
| CTAAACTCTGCAAATGCAGGAA | 22 | GCAA |
| GTGATATCTTTGTCAGTATAAC | 23 | TTGT |
| AAAAAATATACGCTATATCTCT | 24 | ACGC |
| ATCTGTTTTACATAATCCATCT | 25 | ACAT |
| CTGTTTTACATAATCCATCTAT | 26 | ATAA |
| CTATTTTTCTTGATCCATATGC | 27 | TTGA |
| CATATGCTTTTACCTGCAGGCG | 28 | TTAC |

**Table 3. Example Meganuclease Recognition Sequences Downstream of DMD Exon 44**

| Recognition Sequence | SEQ ID NO: | Overhang |
|---|---|---|
| AAATTACTTTTGACTGTTGTTG | 29 | TTGA |
| TGACTGTTGTTGTCATCATTAT | 30 | TTGT |
| TTGTTGTCATCATTATATTACT | 31 | TCAT |
| TTGTCATCATTATATTACTAGA | 32 | TTAT |
| ATCATTATATTACTAGAAAGAA | 33 | TTAC |
| AAAATTATCATAATGATAATAT | 34 | ATAA |
| ATGGACTTTTTGTGTCAGGATG | 35 | TTGT |
| GGACTTTTTGTGTCAGGATGAG | 36 | GTGT |
| GGAGCTGGTTTATCTGATAAAC | 37 | TTAT |
| ATTGAATCTGTGACAGAGGGAA | 38 | GTGA |
| AGGGAAGCATCGTAACAGCAAG | 39 | TCGT |
| GGGCAGTGTGTATTTCGGCTTT | 40 | GTAT |
| TATATTCTATTGACAAAATGCC | 41 | TTGA |
| TAATTGTTGGTACTTATTGACA | 42 | GTAC |
| TGTTGGTACTTATTGACATTTT | 43 | TTAT |
| TTTTATGGTTTATGTTAATAGG | 44 | TTAT |

**Table 4. Example Meganuclease Recognition Sequences Upstream of DMD Exon 45**

| Recognition Sequence | SEQ ID NO: | Overhang |
|---|---|---|
| AGTTTTTTTTTAATACTGTGAC | 45 | TTAA |
| TTTAATACTGTGACTAACCTAT | 46 | GTGA |
| TTTCACCTCTCGTATCCACGAT | 47 | TCGT |
| TCACCTCTCGTATCCACGATCA | 48 | GTAT |
| CTCGTATCCACGATCACTAAGA | 49 | ACGA |
| CCAAATACTTTGTTCATGTTTA | 50 | TTGT |
| GGAACATCCTTGTGGGGACAAG | 51 | TTGT |
| AATTTGCTCTTGAAAAGGTTTC | 52 | TTGA |
| CTAATTGATTTGTAGGACATTA | 53 | TTGT |
| TTCCCTGACACATAAAAGGTGT | 54 | ACAT |
| CCCTGACACATAAAAGGTGTCT | 55 | ATAA |
| CTTTCTGTCTTGTATCCTTTGG | 56 | TTGT |
| ATCCTTTGGATATGGGCATGTC | 57 | ATAT |
| TGGATATGGGCATGTCAGTTTC | 58 | GCAT |
| GATATGGGCATGTCAGTTTCAT | 59 | ATGT |
| GAAATTTTCACATGGAGCTTTT | 60 | ACAT |
| TTTCTTTCTTTGCCAGTACAAC | 61 | TTGC |
| TCTTTGCCAGTACAACTGCATG | 62 | GTAC |
| TTTGGTATCTTACAGGAACTCC | 63 | TTAC |

**Table 5. Example Meganuclease Recognition Sequences Downstream of DMD Exon 45**

| Recognition Sequence | SEQ ID NO: | Overhang |
|---|---|---|
| AAGAATATTTCATGAGAGATTA | 64 | TCAT |
| GAATATTTCATGAGAGATTATA | 65 | ATGA |
| TGAGAGATTATAAGCAGGGTGA | 66 | ATAA |
| AAGGCACTAACATTAAAGAACC | 67 | ACAT |
| TCAACAGCAGTAAAGAAATTTT | 68 | GTAA |
| TTCTTTTTTTCATATACTAAAA | 69 | TCAT |
| CTAAAATATATACTTGTGGCTA | 70 | ATAC |
| TGAATATCTTCAATATATTTTA | 71 | TCAA |
| CAATTATAAATGATTGTTTTGT | 72 | ATGA |
| ATGATTGTTTTGTAGGAAAGAC | 73 | TTGT |
| TCATATTTTGTACAAAATAAAC | 74 | GTAC |

**Table 6. Example Meganuclease Recognition Sequences Upstream of DMD Exon 51**

| Recognition Sequence | SEQ ID NO: | Overhang |
|---|---|---|
| ATACGTGTATTGCTTGTACTAC | 75 | TTGC |
| GTATTGCTTGTACTACTCACTG | 76 | GTAC |
| ACTGAATCTACACAACTGCCCT | 77 | ACAC |
| TGAATCTACACAACTGCCCTTA | 78 | ACAA |
| CAACTGCCCTTATGACATTTAC | 79 | TTAT |
| GGTAAATACATGAAAAATGCTT | 80 | ATGA |
| TTGCCTTGCTTACTGCTTATTG | 81 | TTAC |
| GCTTACTGCTTATTGCTAGTAC | 82 | TTAT |
| TAGTACTGAACAAATGTTAGAA | 83 | ACAA |
| ACTGAACAAATGTTAGAACTGA | 84 | ATGT |
| AAGATTTATTTAATGACTTTGA | 85 | TTAA |
| CAGTATTTCATGTCTAAATAGA | 86 | ATGT |
| GGTTTTTCTTCACTGCTGGCCA | 87 | TCAC |
| CAATCTGAAATAAAAAGAAAAA | 88 | ATAA |
| CTGCTCCCAGTATAAAATACAG | 89 | GTAT |
| AAGAACGTTTCATTGGCTTTGA | 90 | TCAT |
| ACTTCCTATTCAAGGGAATTTT | 91 | TCAA |
| TGTTTTTTCTTGAATAAAAAAA | 92 | TTGA |
| TTTTCTTGAATAAAAAAAAAAT | 93 | ATAA |
| TTGTTTTCTTTACCACTTCCAC | 94 | TTAC |
| ACAATGTATATGATTGTTACTG | 95 | ATGA |
| TGTATATGATTGTTACTGAGAA | 96 | TTGT |
| CTTGTCCAGGCATGAGAATGAG | 97 | GCAT |
| TGTCCAGGCATGAGAATGAGCA | 98 | ATGA |
| AATCGTTTTTTAAAAAATTGTT | 99 | TTAA |
| TTCTACCATGTATTGCTAAACA | 100 | GTAT |
| TACCATGTATTGCTAAACAAAG | 101 | TTGC |
| TATAATGTCATGAATAAGAGTT | 102 | ATGA |
| ATGTCATGAATAAGAGTTTGGC | 103 | ATAA |
| TTTTCCTTTTTGCAAAAACCCA | 104 | TTGC |
| TTCCTTTTTGCAAAAACCCAAA | 105 | GCAA |

**Table 7. Example Meganuclease Recognition Sequences Downstream of DMD Exon 51**

| Recognition Sequence | SEQ ID NO: | Overhang |
|---|---|---|
| AGTTCTTAGGCAACTGTTTCTC | 106 | GCAA |
| TCTCTCTCAGCAAACACATTAC | 107 | GCAA |
| TAAGTATAATCAAGGATATAAA | 108 | TCAA |
| AGTAGCCATACATTAAAAAGGA | 109 | ACAT |
| AGGAAATATACAAAAAAAAAAA | 110 | ACAA |
| AGAAACCTTACAAGAATAGTTG | 111 | ACAA |
| CAAGAATAGTTGTCTCAGTTAA | 112 | TTGT |
| ATCTATTTTATACCAAATAAGT | 113 | ATAC |
| TTATACCAAATAAGTCACTCAA | 114 | ATAA |
| TTTGTTTTGGCACTACGCAGCC | 115 | GCAC |
| TAAGGATAATTGAAAGAGAGCT | 116 | TTGA |
| AGAAAAGTAACAAAACATAAGA | 117 | ACAA |
| TTAAAGTTGGCATTTATGCAAT | 118 | GCAT |
| AGTTGGCATTTATGCAATGCCA | 119 | TTAT |
| AACATGTTTTTAATACAAATAG | 120 | TTAA |
| TACATTGATGTAAATATGGTTT | 121 | GTAA |
| ATATCTTTTATATTTGTGAATG | 122 | ATAT |
| CTTTTATATTTGTGAATGATTA | 123 | TTGT |
| TGTGAATGATTAAGAAAAATAA | 124 | TTAA |
| AATTGTTATACATTAAAGTTTT | 125 | ACAT |
| AAAGTTTTTTCACTTGTAACAG | 126 | TCAC |
| TAACAGCTTTCAAGCCTTTCTA | 127 | TCAA |
| GGTATTTAGGTATTAAAGTACT | 128 | GTAT |
| TACTACCTTTTGAAAAAACAAG | 129 | TTGA |
| GGAATTTCTTTGTAAAATAAAC | 130 | TTGT |
| AACCTGCATTTAAAGGCCTTGA | 131 | TTAA |
| TGAGCTTGAATACAGAAGACCT | 132 | ATAC |
| TGATTGTGGTCAAGCCATCTCT | 133 | TCAA |
| CTATTCTGAGTACAGAGCATAC | 134 | GTAC |

### 2.3 Methods for Delivering and Expressing Nucleases

Treating Duchenne Muscular Dystrophy using the invention requires that a pair of nucleases be expressed in a muscle cell. The nucleases can be delivered as purified protein or as RNA or DNA encoding the nucleases. In one embodiment, the nuclease proteins or mRNA or vector encoding the nucleases are supplied to muscle cells via intramuscular injection (Maltzahn, et al. (2012) Proc Natl Acad Sci U S A. 109:20614-9) or hydrodynamic injection (Taniyama et al. (2012) Curr Top Med Chem. 12:1630-7; Hegge, et al. (2010) Hum Gene Ther. 21:829-42). To facilitate cellular uptake, the proteins or nucleic acid(s) can be coupled to a cell penetrating peptide to facilitate uptake by muscle cells. Examples of cell pentrating peptides known in the art include poly-arginine (Jearawiriyapaisarn, et al. (2008) Mol Ther. 16:1624-9), TAT peptide from the HIV virus (Hudecz et al. (2005), Med. Res. Rev. 25: 679-736), MPG (Simeoni, et al. (2003) Nucleic Acids Res. 31:2717-2724), Pep-1 (Deshayes et al. (2004) Biochemistry 43: 7698-7706, and HSV-1 VP-22 (Deshayes et al. (2005) Cell Mol Life Sci. 62:1839-49. Alternatively, cell penetration can be facilitated by liposome encapsulation (see, e.g., Lipofectamine^{™}, Life Technologies Corp., Carlsbad, CA). The liposome formulation can be used to facilitate lipid bilayer fusion with a target cell, thereby allowing the contents of the liposome or proteins associated with its surface to be brought into the cell.

In some embodiments, the genes encoding a pair of nucleases are delivered using a viral vector. Such vectors are known in the art and include lentiviral vectors, adenoviral vectors, and adeno-associated virus (AAV) vectors (reviewed in Vannucci, et al. (2013 New Microbiol. 36:1-22). In some embodiments, the viral vectors are injected directly into muscle tissue. In alternative embodiments, the viral vectors are delivered systemically. Example 3 describes a preferred embodiment in which the muscle is injected with a recombinant AAV virus encoding a pair of single-chain meganucleases. It is known in the art that different AAV vectors tend to localize to different tissues. Muscle-tropic AAV serotypes include AAV1, AAV9, and AAV2.5 (Bowles, et al. (2012) Mol Ther. 20:443-55). Thuse, these serotypes are preferred for the delivery of nucleases to muscle tissue.

If the nuclease genes are delivered in DNA form (e.g. plasmid) and/or via a viral vector (*e.g*. AAV) they must be operably linked to a promoter. In some embodiments, this can be a viral promoter such as endogenous promoters from the viral vector *(e.g.* the LTR of a lentiviral vector) or the well-known cytomegalovirus- or SV40 virus-early promoters. In a preferred embodiment, the nuclease genes are operably linked to a promoter that drives gene expression preferentially in muscle cells. Examples of muscle-specific promoters include C5-12 (Liu, et al. (2004) Hum Gene Ther. 15:783-92), the muscle-specific creatine kinase (MCK) promoter (Yuasa, et al. (2002) Gene Ther. 9:1576-88), or the smooth muscle 22 (SM22) promoter (Haase, et al. (2013) BMC Biotechnol. 13:49-54). In some embodiments, the nuclease genes are under the control of two separate promoters. In alternative embodiments, the genes are under the control of a single promoter and are separated by an internal-ribosome entry site (IRES) or a 2A peptide sequence (Szymczak and Vignali (2005) Expert Opin Biol Ther. 5:627-38).

It is envisioned that a single treatment will permanently delete exons from a percentage of patient cells. In preferred embodiments, these cells will be myoblasts or other muscle precurser cells that are capable of replicating and giving rise to whole muscle fibers that express functional (or semi-functional) dystrophin. If the frequency of exon deletion is low, however, it may be necessary to perform multiple treatments on each patient such as multiple rounds of intramuscular injections.

### EXAMPLES

This invention is further illustrated by the following examples, which should not be construed as limiting. Those skilled in the art will recognize, or be able to ascertain, using no more than routine experimentation, numerous equivalents to the specific substances and procedures described herein. Such equivalents are intended to be encompassed in the scope of the claims that follow the examples below.

### EXAMPLE 1

### Deletion of DMD Exon 44 Using a Pair of Engineered, Single-Chain Meganucleases

### 1. Meganucleases that recognize SEQ ID NO: 19 and SEQ ID NO: 42

An engineered meganuclease (SEQ ID NO: 135) was produced which recognizes and cleaves SEQ ID NO: 19. This meganuclease is called "DYS-1/2". A second engineered meganuclease (SEQ ID NO: 136) was produced which recognizes and cleaves SEQ ID NO: 42. This meganuclease is called "DYS-3/4" (Figure 3A). Each meganuclease comprises an N-terminal nuclease-localization signal derived from SV40, a first meganuclease subunit, a linker sequence, and a second meganuclease subunit.

### 2. Deletion of DMD Exon 44 in HEK-293 cells

Human embryonic kidney (HEK-293) cells were co-transfected with mRNA encoding DYS-1/2 and DYS-3/4. mRNA was prepared by first producing a PCR template for an *in vitro* transcription reaction (SEQ ID NO: 139 and SEQ ID NO: 140. Each PCR product included a T7 promoter and 609 bp of vector sequence downstream of the meganuclease gene. The PCR product was gel purified to ensure a single template. Capped (m7G) RNA was generated using the RiboMAX T7 kit (Promega) according to the manufacturer's instructions and. Ribo m7G cap analog (Promega) was included in the reaction and 0.5 ug of the purified meganuclease PCR product served as the DNA template. Capped RNA was purified using the SV Total RNA Isolation System (Promega) according to the manufacturer's instructions.

1.5x10⁶ HEK-293 cells were nucleofected with 1.5x10¹² copies of DYS-1/2 mRNA and 1.5x10¹² copies of DYS-3/4 mRNA (2x10⁶ copies/cell) using an Amaxa Nucleofector II device (Lonza) according to the manufacturer's instructions. 48 hours post-transfection, genomic DNA was isolated from the cells using a FlexiGene kit (Qiagen) according to the manufacturer's instructions. The genomic DNA was then subjected to PCR using primers flanking the DYS-1/2 and DYS-3/4 cut sites (SEQ ID NO: 141 and SEQ ID NO:142). When PCR products were resolved by agarose gel electrophoresis, it was apparent that cells co-expressing DYS-1/2 and DYS-3/4 yielded two PCR products with apparent lengths of 1079 basepairs and 233 basepairs whereas genomic DNA from untransfected HEK-293 cells yielded only the larger product (Figure 3B). The larger product is consistent with the expected size of a PCR fragment from cells with intact *DMD* Exon 44. The smaller product is consistent with the expected size of a PCR fragment from cells in which Exon 44 has been excised from the *DMD* gene.

The smaller PCR product was isolated from the gel and cloned into a bacterial plasmid (pUC-19) for sequence analysis. Three plasmid clones were sequenced, all of which were found to have Exon 44 deleted (Figure 3C). Surprisingly, two of the three plasmids carried PCR products from cells in which the deletion consisted precisely of the region intervening the expected DYS-1/2 and DYS-3/4-induced DNA breaks. It appears that the two meganucleases cleaved their intended recognition sites, leaving compatible 5'-GTAC-3' overhangs, the intervening fragment comprising Exon 44 was lost, and the two chromosome ends were then re-ligated. The third plasmid clone carried a PCR product from a cell in which the region intervening the two cleavage sites was excised along with 10 additional bases.

### 3. Conclusions

We have demonstrated that it is possible to use a pair of engineered single-chain meganucleases to excise a fragment from the human genome in a cultured cell line. The DNA removal and repair process appears to have proceeded via a mechanism that involves the 3' overhangs produced by the nucleases, suggesting that the process is more efficient when the overhangs are complementary and able to anneal to one another.

### EXAMPLE 2

### Deletion of DMD Exon 45 Using a Pair of Engineered, Single-Chain Meganucleases

### 1. Meganucleases that recognize SEQ ID NO: 62 and SEQ ID NO: 74

An engineered meganuclease (SEQ ID NO: 137) was produced which recognizes and cleaves SEQ ID NO: 62. This meganuclease is called "DYS-5/6". A second engineered meganuclease (SEQ ID NO: 138) was produced which recognizes and cleaves SEQ ID NO: 74. This meganuclease is called "DYS-7/8" (Figure 4A). Each meganuclease comprises an N-terminal nuclease-localization signal derived from SV40, a first meganuclease subunit, a linker sequence, and a second meganuclease subunit.

### 2. Deletion of DMD Exon 45 in HEK-293 cells

Human embryonic kidney (HEK-293) cells were co-transfected with mRNA encoding DYS-5/6 and DYS-7/8. mRNA was prepared by first producing a PCR template for an *in vitro* transcription reaction (SEQ ID NO: 143(20) and SEQ ID NO: 144(21). Each PCR product included a T7 promoter and 609 bp of vector sequence downstream of the meganuclease gene. The PCR product was gel purified to ensure a single template. Capped (m7G) RNA was generated using the RiboMAX T7 kit (Promega) according to the manufacturer's instructions and. Ribo m7G cap analog (Promega) was included in the reaction and 0.5 ug of the purified meganuclease PCR product served as the DNA template. Capped RNA was purified using the SV Total RNA Isolation System (Promega) according to the manufacturer's instructions.

1.5x10⁶ HEK-293 cells were nucleofected with 1.5x10¹² copies of DYS-5/6 mRNA and 1.5x10¹² copies of DYS-7/8 mRNA (2x10⁶ copies/cell) using an Amaxa Nucleofector II device (Lonza) according to the manufacturer's instructions. 48 hours post-transfection, genomic DNA was isolated from the cells using a FlexiGene kit (Qiagen) according to the manufacturer's instructions. The genomic DNA was then subjected to PCR using primers flanking the DYS-5/6 and DYS-7/8 cut sites (SEQ ID NO: 145 and SEQ ID NO:146). When PCR products were resolved by agarose gel electrophoresis, it was apparent that cells co-expressing DYS-5/6 and DYS-7/8 yielded two PCR products with apparent lengths of 1384 basepairs and 161 basepairs whereas genomic DNA from untransfected HEK-293 cells yielded only the larger product (Figure 4B). The larger product is consistent with the expected size of a PCR fragment from cells with intact *DMD* Exon 45. The smaller product is consistent with the expected size of a PCR fragment from cells in which Exon 45 has been excised from the *DMD* gene.

The smaller PCR product was isolated from the gel and cloned into a bacterial plasmid (pUC-19) for sequence analysis. 16 plasmid clones were sequenced, all of which were found to have Exon 45 deleted (Figure 4C). Surprisingly, 14 of the 16 plasmids carried PCR products from cells in which the deletion consisted precisely of the region intervening the expected DYS-5/6 and DYS-7/8-induced DNA breaks. It appears that the two meganucleases cleaved their intended recognition sites, leaving compatible 5'-GTAC-3' overhangs, the intervening fragment comprising Exon 45 was lost, and the two chromosome ends were then re-ligated. The two remaining plasmid clones carried PCR product from cells in which the region intervening the two cleavage sites was excised along with 36 additional bases.

### 3. Conclusions

We have demonstrated that it is possible to use a pair of engineered single-chain meganucleases to excise a fragment from the human genome in a cultured cell line. The DNA removal and repair process appears to have proceeded via a mechanism that involves the 3' overhangs produced by the nucleases, suggesting that the process is more efficient when the overhangs are complementary and able to anneal to one another.

### EXAMPLE 3

### Deletion of DMD Exon 23 in a mouse using AAV-delivered meganucleases

### 1. Development of nucleases to delete mouse DMD Exon 23

The standard mouse model of DMD is the mdx mouse, which has a point mutation in Exon 23 that introduces a premature stop codon (Sicinski et al. (1989) Science. 244:1578-80). In the mouse, *DMD* Exon 23 is 213 basepairs, equivalent to 71 amino acids. Thus, we reasoned that it should be possible to delete Exon 23 in its entirety and thereby remove the stop codon while maintaining the reading frame of the *DMD* gene. To this end, we developed a pair of single-chain meganucleases called "MDX-1/2" (SEQ ID NO: 147) and "MDX-13/14" (SEQ ID NO: 148). The former recognizes a DNA sequence upstream of mouse *DMD* Exon 23 (SEQ ID NO: 149) while the latter recognizes a DNA sequence downstream of mouse *DMD* Exon 23 (SEQ ID NO: 150). The nucleases were tested, initially, using a reporter assay called "iGFFP" in CHO cells as shown in Figure 5. Both nucleases were found to efficiently cut their intended DNA sites using this assay.

### 2. Deletion of mouse DMD Exon 23 in mouse myoblast cells

A mouse myoblast cell line (C2C12) was co-transfected with *in* vitro transcribed mRNA encoding the MDX-1/2 and MDX-13/14 nucleases. mRNA was produced using the RiboMAX T7 kit from Promega. 1e6 C2C12 cells were Nucleofected with a total of 2e6 copies/cell of mRNA encoding each MDX enzyme pairs (1e6 copies of each mRNA) using an Amaxa 2b device and the B-032 program. After 96 hours, cells were cloned by limiting dilution in 96-well plates. After approximately 2 weeks growth, cells were harvested and genomic DNA was isolated using a FlexiGene kit from Qiagen. A PCR product was then generated for each clone using a forward primer in *DMD* Intron 22 (SEQ ID NO: 151) and a reverse primer in Intron 23 (SEQ ID NO: 152). 60 of the PCR products were then cloned and sequenced. 20 of the sequences had deletions consistent with meganuclease-induced cleavage of the *DMD* gene followed by mutagenic DNA repair (Figure 6, SEQ ID NO:153-172). 11 of the sequences were missing at least a portion of the MDX-1/2 and MDX-13/14 recognition sites, as well as Exon 23 (SEQ ID NO:153-163). These sequences were likely derived from cells in which both nucleases cut their intended sites and the intervening sequence was deleted. 4 of the sequences were missing Exon 23 but had an intact MDX-1/2 recognition sequence (SEQ ID NO:164-167). These appear to be due to DNA cleavage by MDX-13/14 alone followed by the deletion of a large amount of sequence. Five of the sequences had an intact MDX-1/2 recognition site and all or a portion of Exon 23 but were missing all or a portion of the MDX-13/14 recognition site (SEQ ID NO:168-172). These sequences appear to be due to DNA cleavage by MDX-13/14 alone followed by the deletion of a smaller amount of sequence insufficient to eliminate all of Exon 23. In stark contrast to the experiments in Examples 1 and 2, we did not obtain a consistent DNA sequence following the deletion of *DMD* Exon 23 in the mouse cells. This is likely because the two MDX meganucleases do not generate DNA breaks with compatible 3' overhangs. MDX-1/2 generates an overhang with the sequence 5'-GTGA-3' and MDX-13/14 generates an overhang with the sequence 5'-ACAC-3'. Thus, we conclude that the consistent sequence results obtained in Examples 1 and 2 are due to the compatibility of the 3' overhangs generated by the pair of meganucleases.

### 3. Generation of recombinant AAV vectors for delivery of a pair of engineered nucleases.

To produce AAV vectors for simultaneous delivery of MDX-1/2 and MDX-13/14 genes, we first produced a "packaging" plasmid called "pAAV-MDX" (Fig. 7, SEQ ID NO. 173) comprising a pair of inverted terminal repeat (ITR) sequences from AAV2, as well as the gene coding sequences for the MDX-1/2 and MDX-13/14 meganucleases, each under the control of a CMV Early promoter. This vector was used to produce recombinant AAV2 virus by co-transfection of HEK-293 cells with an Ad helper plasmid according to the method of Xiao, *et al* (Xiao, et al. (1998) J. Virology 72:2224-2232). Virus was then isolated by cesium-chloride gradient centrifugation as described by Grieger and Samulski (Grieger and Samulski (2012) Methods Enzymol. 507:229-254). To confirm that the resulting virus particles were infectious and capable of expressing both engineered meganucleases, they were added to cultured iGFFP CHO cells carrying reporter cassettes for either MDX-1/2 or MDX-13/14 (see Figure 5A). The addition of recombinant virus particles to the CHO line carrying a reporter cassette for MDX-1/2 resulted in GFP gene expression in 7.1% of the cells. The addition of virus to the CHO line carrying a reporter for MDX-13/14 resulted in GFP gene expression in 10.2% of cells. Thus, we conclude that the virus was able to transduce CHO cells and that transduced cells expressed both nucleases.

### 4. Deletion of DMD Exon 23 in mouse muscle following AAV delivery of a pair of meganuclease genes.

Recombinant AAV1 virus particles carrying the MDX-1/2 and MDX-13/14 genes were produced as described above. Three hindlimb TA muscles from a pair of *mdx* mice were injected with virus as described in Xiao, *et al* (Xiao, et al. (1998) J. Virology 72:2224-2232). One muscle from one mouse was not injected as a negative control. Muscles from the two mice were harvested at 4 days or 7 days post-injection and genomic DNA was isolated from the muscle tissue. The genomic region surrounding *DMD* Exon 23 was amplified by PCR using a first primer pair (SEQ ID NO: 151 and SEQ ID NO: 152). This reaction was then used to template a second PCR reaction using a "nested" primer pair (SEQ ID NO: 174 and SEQ ID NO: 175) to eliminate non-specific PCR products. PCR products were then visualized on an agarose gel and it was found that genomic DNA from the three AAV1 injected muscles, but not the un-injected control muscle, yielded smaller PCR products that were consistent in size with the product expected following deletion of *DMD* Exon 23 by the MDX-1/2 and MDX-13/14 meganucleases. The smaller PCR products were then cloned and sequenced. Three unique sequences were obtained, each of which comprised a portion of the mouse *DMD* gene including part of Intron 22 and Intron 23 but lacking Exon 23 and all of the sequence intervening the cut sites for the MDX-1/2 and MDX-13/14 meganucleases (SEQ ID NO: 176-178). Thus, we have demonstrated that a pair of meganucleases delivered by AAV can be used to delete a portion of the *DMD* gene *in vivo* from mouse muscle.

### 5. Conclusions

We have demonstrated that the genes encoding a pair of engineered single-chain meganucleases can be delivered to cells and organisms using recombinant AAV vectors and that meganucleases so delivered are able to cleave genomic DNA in the cell and delete fragments of DNA from the genome. We have further demonstrated that a pair of meganuclease-induced DNA breaks that do not generate compatible overhangs will not re-ligate to yield a defined sequence outcome following removal of the intervening sequence. Thus, for therapeutic applications in which a defined sequence outcome is desirable, it is preferable to use a pair of nucleases that generate identical overhangs.

### SEQUENCE LISTING

SEQ ID NO: 1 (wild-type I-CreI, Genbank Accession # PO5725) 
1 MNTKYNKEFL LYLAGFVDGD GSIIAQIKPN QSYKFKHQLS LAFQVTQKTQ RRWFLDKLVD
61 EIGVGYVRDR GSVSDYILSE IKPLHNFLTQ LQPFLKLKQK QANLVLKIIW RLPSAKESPD
121 KFLEVCTWVD QIAALNDSKT RKTTSETVRA VLDSLSEKKK SSP
SEQ ID NO: 135 (DYS-1/2) 
1 MAPKKKRKVH MNTKYNKEFL LYLAGFVDGD GSIYAWISPS QTCKFKHRLM LRFIVSQKTQ
61 RRWFLDKLVD EIGVGYVQDC GSVSEYRLSE IKPLHNFLTQ LQPFLKLKQK QANLVLKIIE
121 QLPSAKESPD KFLEVCTWVD QIAALNDSKT RKTTSETVRA VLDSLPGSVG GLSPSQASSA
181 ASSASSSPGS GISEALRAGA GSGTGYNKEF LLYLAGFVDG DGSIYACILP TQRQKFKHGL
241 TLYFRVTQKT QRRWFLDKLV DEIGVGYVLD FGSVSCYSLS QIKPLHNFLT QLQPFLKLKQ
301 KQANLVLKII EQLPSAKESP DKFLEVCTWV DQIAALNDSK TRKTTSETVR AVLDSLSEKK
361 KSSP
SEQ ID NO: 136 (DYS-3/4) 
1 MAPKKKRKVH MNTKYNKEFL LYLAGFVDGD GSIFASIRPR QTSKFKHALA LFFVVGQKTQ
61 RRWFLDKLVD EIGVGYVYDR GSVSVYQLSQ IKPLHNFLTQ LQPFLKLKQK QANLVLKIIE
121 QLPSAKESPD KFLEVCTWVD QIAALNDSKT RKTTSETVRA VLDSLPGSVG GLSPSQASSA
181 ASSASSSPGS GISEALRAGA GSGTGYNKEF LLYLAGFVDG DGSIIACIRP HQAYKFKHQL
241 CLSFCVYQKT QRRWFLDKLV DEIGVGYVTD AGSVSSYRLS EIKPLHNFLT QLQPFLKLKQ
301 KQANLVLKII EQLPSAKESP DKFLEVCTWV DQIAALNDSK TRKTTSETVR AVLDSLSEKK
361 KSSP
SEQ ID NO: 137 (DYS-5/6) 
1 MAPKKKRKVH MNTKYNKEFL LYLAGFVDGD GSIFACIQPD QRAKFKHTLR LSFEVGQKTQ
61 RRWFLDKLVD EIGVGYVNDS GSVSKYRLSQ IKPLHNFLTQ LQPFLKLKQK QANLVLKIIE
121 QLPSAKESPD KFLEVCTWVD QIAALNDSKT RKTTSETVRA VLDSLPGSVG GLSPSQASSA
181 ASSASSSPGS GISEALRAGA GSGTGYNKEF LLYLAGFVDG DGSIYATIQP TQCAKFKHQL
241 TLRFSVSQKT QRRWFLDKLV DEIGVGYVCD KGSVSEYMLS EIKPLHNFLT QLQPFLKLKQ
301 KQANLVLKII EQLPSAKESP DKFLEVCTWV DQIAALNDSK TRKTTSETVR AVLDSLSEKK
361 KSSP
SEQ ID NO: 138 (DYS-7/8) 
1 MAPKKKRKVH MNTKYNKEFL LYLAGFVDGD GSIYACILPV QRCKFKHGLS LRFMVSQKTQ
61 RRWFLDKLVD EIGVGYVYDC GSVSEYRLSE IKPLHNFLTQ LQPFLKLKQK QANLVLKIIE
121 QLPSAKESPD KFLEVCTWVD QIAALNDSKT RKTTSETVRA VLDSLPGSVG GLSPSQASSA
181 ASSASSSPGS GISEALRAGA GSGTGYNKEF LLYLAGFVDG DGSIFASIVP DQRSKFKHGL
241 ALRFNVVQKT QRRWFLDKLV DEIGVGYVYD QGSVSEYRLS EIKPLHNFLT QLQPFLKLKQ
301 KQANLVLKII EQLPSAKESP DKFLEVCTWV DQIAALNDSK TRKTTSETVR AVLDSLSEKK
361 KSSP
SEQ ID NO: 139 (DYS-1/2 PCR Template for mRNA) 
1 CACAGGTGTC CACTCCCAGT TCAATTACAG CTCTTAAGGC TAGAGTACTT AATACGACTC
61 ACTATAGGCT AGCCTCGAGC CGCCACCATG GCACCGAAGA AGAAGCGCAA GGTGCATATG
121 AATACAAAAT ATAATAAAGA GTTCTTACTC TACTTAGCAG GGTTTGTAGA CGGTGACGGT
181 TCCATCTATG CCTGGATCAG TCCTTCGCAA ACGTGTAAGT TCAAGCACAG GCTGATGCTC
241 CGGTTCATTG TCTCGCAGAA GACACAGCGC CGTTGGTTCC TCGACAAGCT GGTGGACGAG
301 ATCGGTGTGG GTTACGTGCA GGACTGTGGC AGCGTCTCCG AGTACCGGCT GTCCGAGATC
361 AAGCCTTTGC ATAATTTTTT AACACAACTA CAACCTTTTC TAAAACTAAA ACAAAAACAA
421 GCAAATTTAG TTTTAAAAAT TATTGAACAA CTTCCGTCAG CAAAAGAATC CCCGGACAAA
481 TTCTTAGAAG TTTGTACATG GGTGGATCAA ATTGCAGCTC TGAATGATTC GAAGACGCGT
541 AAAACAACTT CTGAAACCGT TCGTGCTGTG CTAGACAGTT TACCAGGATC CGTGGGAGGT
601 CTATCGCCAT CTCAGGCATC CAGCGCCGCA TCCTCGGCTT CCTCAAGCCC GGGTTCAGGG
661 ATCTCCGAAG CACTCAGAGC TGGAGCAGGT TCCGGCACTG GATACAACAA GGAATTCCTG
721 CTCTACCTGG CGGGCTTCGT CGACGGGGAC GGCTCCATCT ATGCCTGTAT CCTTCCGACT
 
781 CAGCGTCAGA AGTTCAAGCA CGGGCTGACG CTCTATTTCC GGGTCACTCA GAAGACACAG
841 CGCCGTTGGT TCCTCGACAA GCTGGTGGAC GAGATCGGTG TGGGTTACGT GCTGGACTTT
901 GGCAGCGTCT CCTGTTACTC TCTGTCCCAG ATCAAGCCTC TGCACAACTT CCTGACCCAG
961 CTCCAGCCCT TCCTGAAGCT CAAGCAGAAG CAGGCCAACC TCGTGCTGAA GATCATCGAG
1021 CAGCTGCCCT CCGCCAAGGA ATCCCCGGAC AAGTTCCTGG AGGTGTGCAC CTGGGTGGAC
1081 CAGATCGCCG CTCTGAACGA CTCCAAGACC CGCAAGACCA CTTCCGAAAC CGTCCGCGCC
1141 GTTCTAGACA GTCTCTCCGA GAAGAAGAAG TCGTCCCCCT AAACAGTCTC TCCGAGAAGA
1201 AGAAGTCGTC CCCCTAGCGG CCGCTTCGAG CAGACATGAT AAGATACATT GATGAGTTTG
1261 GACAAACCAC AACTAGAATG CAGTGAAAAA AATGCTTTAT TTGTGAAATT TGTGATGCTA
1321 TTGCTTTATT TGTAACCATT ATAAGCTGCA ATAAACAAGT TAACAACAAC AATTGCATTC
1381 ATTTTATGTT TCAGGTTCAG GGGGAGATGT GGGAGGTTTT TTAAAGCAAG TAAAACCTCT
1441 ACAAATGTGG TAAAATCGAT AAGATCTTGA TCCGGGCTGG CGTAATAGCG AAGAGGCCCG
1501 CACCGATCGC CCTTCCCAAC AGTTGCGCAG CCTGAATGGC GAATGGACGC GCCCTGTAGC
1561 GGCGCATTAA GCGCGGCGGG TGTGGTGGTT ACGCGCAGCG TGACCGCTAC ACTTGCCAGC
1621 GCCCTAGCGC CCGCTCCTTT CGCTTTCTTC CCTTCCTTTC TCGCCACGTT CGCCGGCTTT
1681 CCCCGTCAAG CTCTAAATCG GGGGCTCCCT TTAGGGTTCC GATTTAGTGC TTTACGGCAC
1741 CTCGACCCCA AAAAACTTGA TTAGGGTGAT GGTTCACGTA GTGGGCCATC G
SEQ ID NO: 140 (DYS-3/4 PCR Template for mRNA) 
1 CACAGGTGTC CACTCCCAGT TCAATTACAG CTCTTAAGGC TAGAGTACTT AATACGACTC
61 ACTATAGGCT AGCCTCGAGC CGCCACCATG GCACCGAAGA AGAAGCGCAA GGTGCATATG
121 AATACAAAAT ATAATAAAGA GTTCTTACTC TACTTAGCAG GGTTTGTAGA CGGTGACGGT
181 TCCATCTTTG CCTCTATCCG GCCTCGGCAA ACGAGTAAGT TCAAGCACGC GCTGGCTCTC
241 TTTTTCGTGG TCGGGCAGAA GACACAGCGC CGTTGGTTCC TCGACAAGCT GGTGGACGAG
301 ATCGGTGTGG GTTACGTGTA TGACCGTGGC AGCGTCTCCG TGTACCAGCT GTCCCAGATC
361 AAGCCTTTGC ATAATTTTTT AACACAACTA CAACCTTTTC TAAAACTAAA ACAAAAACAA
421 GCAAATTTAG TTTTAAAAAT TATTGAACAA CTTCCGTCAG CAAAAGAATC CCCGGACAAA
481 TTCTTAGAAG TTTGTACATG GGTGGATCAA ATTGCAGCTC TGAATGATTC GAAGACGCGT
541 AAAACAACTT CTGAAACCGT TCGTGCTGTG CTAGACAGTT TACCAGGATC CGTGGGAGGT
601 CTATCGCCAT CTCAGGCATC CAGCGCCGCA TCCTCGGCTT CCTCAAGCCC GGGTTCAGGG
661 ATCTCCGAAG CACTCAGAGC TGGAGCAGGT TCCGGCACTG GATACAACAA GGAATTCCTG
721 CTCTACCTGG CGGGCTTCGT CGACGGGGAC GGCTCCATCA TTGCCTGTAT CCGGCCTCAT
781 CAAGCTTATA AGTTCAAGCA CCAGCTGTGT CTCTCTTTCT GTGTCTATCA GAAGACACAG
841 CGCCGTTGGT TCCTCGACAA GCTGGTGGAC GAGATCGGTG TGGGTTACGT GACGGACGCT
901 GGCAGCGTCT CCTCTTACCG GCTGTCCGAG ATCAAGCCTC TGCACAACTT CCTGACCCAG
961 CTCCAGCCCT TCCTGAAGCT CAAGCAGAAG CAGGCCAACC TCGTGCTGAA GATCATCGAG
1021 CAGCTGCCCT CCGCCAAGGA ATCCCCGGAC AAGTTCCTGG AGGTGTGCAC CTGGGTGGAC
1081 CAGATCGCCG CTCTGAACGA CTCCAAGACC CGCAAGACCA CTTCCGAAAC CGTCCGCGCC
1141 GTTCTAGACA GTCTCTCCGA GAAGAAGAAG TCGTCCCCCT AAACAGTCTC TCCGAGAAGA
1201 AGAAGTCGTC CCCCTAGCGG CCGCTTCGAG CAGACATGAT AAGATACATT GATGAGTTTG
1261 GACAAACCAC AACTAGAATG CAGTGAAAAA AATGCTTTAT TTGTGAAATT TGTGATGCTA
1321 TTGCTTTATT TGTAACCATT ATAAGCTGCA ATAAACAAGT TAACAACAAC AATTGCATTC
1381 ATTTTATGTT TCAGGTTCAG GGGGAGATGT GGGAGGTTTT TTAAAGCAAG TAAAACCTCT
1441 ACAAATGTGG TAAAATCGAT AAGATCTTGA TCCGGGCTGG CGTAATAGCG AAGAGGCCCG
1501 CACCGATCGC CCTTCCCAAC AGTTGCGCAG CCTGAATGGC GAATGGACGC GCCCTGTAGC
1561 GGCGCATTAA GCGCGGCGGG TGTGGTGGTT ACGCGCAGCG TGACCGCTAC ACTTGCCAGC
1621 GCCCTAGCGC CCGCTCCTTT CGCTTTCTTC CCTTCCTTTC TCGCCACGTT CGCCGGCTTT
1681 CCCCGTCAAG CTCTAAATCG GGGGCTCCCT TTAGGGTTCC GATTTAGTGC TTTACGGCAC
1741 CTCGACCCCA AAAAACTTGA TTAGGGTGAT GGTTCACGTA GTGGGCCATC G
SEQ ID NO: 141 (Exon 44 Forward PCR primer)
   1 GAAAGAAAAT GCCAATAGTC CAAAATAGTT G
SEQ ID NO: 142 (Exon 44 Reverse PCR primer)
   1 CATATTCAAA GGACACCACA AGTTG
SEQ ID NO: 143 DYS-5/6 PCR Template for mRNA) 1 CACAGGTGTC CACTCCCAGT TCAATTACAG CTCTTAAGGC TAGAGTACTT AATACGACTC 
61 ACTATAGGCT AGCCTCGAGC CGCCACCATG GCACCGAAGA AGAAGCGCAA GGTGCATATG
121 AATACAAAAT ATAATAAAGA GTTCTTACTC TACTTAGCAG GGTTTGTAGA CGGTGACGGT
181 TCCATCTTTG CCTGTATCCA GCCTGATCAA AGGGCGAAGT TCAAGCACAC GCTGCGGCTC
241 TCTTTCGAGG TCGGGCAGAA GACACAGCGC CGTTGGTTCC TCGACAAGCT GGTGGACGAG
301 ATCGGTGTGG GTTACGTGAA TGACTCTGGC AGCGTCTCCA AGTACAGGCT GTCCCAGATC
361 AAGCCTTTGC ATAATTTTTT AACACAACTA CAACCTTTTC TAAAACTAAA ACAAAAACAA
421 GCAAATTTAG TTTTAAAAAT TATTGAACAA CTTCCGTCAG CAAAAGAATC CCCGGACAAA
481 TTCTTAGAAG TTTGTACATG GGTGGATCAA ATTGCAGCTC TGAATGATTC GAAGACGCGT
541 AAAACAACTT CTGAAACCGT TCGTGCTGTG CTAGACAGTT TACCAGGATC CGTGGGAGGT
601 CTATCGCCAT CTCAGGCATC CAGCGCCGCA TCCTCGGCTT CCTCAAGCCC GGGTTCAGGG
661 ATCTCCGAAG CACTCAGAGC TGGAGCAGGT TCCGGCACTG GATACAACAA GGAATTCCTG
721 CTCTACCTGG CGGGCTTCGT CGACGGGGAC GGCTCCATCT ATGCCACTAT CCAGCCTACT
781 CAATGTGCGA AGTTCAAGCA CCAGCTGACT CTCCGTTTCT CGGTCTCTCA GAAGACACAG
841 CGCCGTTGGT TCCTCGACAA GCTGGTGGAC GAGATCGGTG TGGGTTACGT GTGTGACAAG
901 GGCAGCGTCT CCGAGTACAT GCTGTCCGAG ATCAAGCCTC TGCACAACTT CCTGACCCAG
961 CTCCAGCCCT TCCTGAAGCT CAAGCAGAAG CAGGCCAACC TCGTGCTGAA GATCATCGAG
1021 CAGCTGCCCT CCGCCAAGGA ATCCCCGGAC AAGTTCCTGG AGGTGTGCAC CTGGGTGGAC
1081 CAGATCGCCG CTCTGAACGA CTCCAAGACC CGCAAGACCA CTTCCGAAAC CGTCCGCGCC
1141 GTTCTAGACA GTCTCTCCGA GAAGAAGAAG TCGTCCCCCT AAACAGTCTC TCCGAGAAGA
1201 AGAAGTCGTC CCCCTAGCGG CCGCTTCGAG CAGACATGAT AAGATACATT GATGAGTTTG
1261 GACAAACCAC AACTAGAATG CAGTGAAAAA AATGCTTTAT TTGTGAAATT TGTGATGCTA
1321 TTGCTTTATT TGTAACCATT ATAAGCTGCA ATAAACAAGT TAACAACAAC AATTGCATTC
1381 ATTTTATGTT TCAGGTTCAG GGGGAGATGT GGGAGGTTTT TTAAAGCAAG TAAAACCTCT
1441 ACAAATGTGG TAAAATCGAT AAGATCTTGA TCCGGGCTGG CGTAATAGCG AAGAGGCCCG
1501 CACCGATCGC CCTTCCCAAC AGTTGCGCAG CCTGAATGGC GAATGGACGC GCCCTGTAGC
1561 GGCGCATTAA GCGCGGCGGG TGTGGTGGTT ACGCGCAGCG TGACCGCTAC ACTTGCCAGC
1621 GCCCTAGCGC CCGCTCCTTT CGCTTTCTTC CCTTCCTTTC TCGCCACGTT CGCCGGCTTT
1681 CCCCGTCAAG CTCTAAATCG GGGGCTCCCT TTAGGGTTCC GATTTAGTGC TTTACGGCAC
1741 CTCGACCCCA AAAAACTTGA TTAGGGTGAT GGTTCACGTA GTGGGCCATC G
SEQ ID NO: 144 (DYS-7/8 PCR Template for mRNA) 
1 CACAGGTGTC CACTCCCAGT TCAATTACAG CTCTTAAGGC TAGAGTACTT AATACGACTC
61 ACTATAGGCT AGCCTCGAGC CGCCACCATG GCACCGAAGA AGAAGCGCAA GGTGCATATG
121 AATACAAAAT ATAATAAAGA GTTCTTACTC TACTTAGCAG GGTTTGTAGA CGGTGACGGT
181 TCCATCTATG CCTGTATCTT GCCGGTGCAG CGTTGTAAGT TCAAGCACGG GCTGTCTCTC
241 CGATTCATGG TCAGTCAGAA GACACAGCGC CGTTGGTTCC TCGACAAGCT GGTGGACGAG
301 ATCGGTGTGG GTTACGTGTA TGACTGTGGC AGCGTCTCCG AGTACAGGCT GTCCGAGATC
361 AAGCCTTTGC ATAATTTTTT AACACAACTA CAACCTTTTC TAAAACTAAA ACAAAAACAA
421 GCAAATTTAG TTTTAAAAAT TATTGAACAA CTTCCGTCAG CAAAAGAATC CCCGGACAAA
481 TTCTTAGAAG TTTGTACATG GGTGGATCAA ATTGCAGCTC TGAATGATTC GAAGACGCGT
541 AAAACAACTT CTGAAACCGT TCGTGCTGTG CTAGACAGTT TACCAGGATC CGTGGGAGGT
601 CTATCGCCAT CTCAGGCATC CAGCGCCGCA TCCTCGGCTT CCTCAAGCCC GGGTTCAGGG
661 ATCTCCGAAG CACTCAGAGC TGGAGCAGGT TCCGGCACTG GATACAACAA GGAATTCCTG
721 CTCTACCTGG CGGGCTTCGT CGACGGGGAC GGCTCCATCT TTGCCTCTAT CGTGCCGGAT
781 CAGCGTAGTA AGTTCAAGCA CGGTCTGGCT CTCAGGTTCA ATGTCGTTCA GAAGACACAG
841 CGCCGTTGGT TCCTCGACAA GCTGGTGGAC GAGATCGGTG TGGGTTACGT GTATGACCAG
901 GGCAGCGTCT CCGAGTACAG GCTGTCCGAG ATCAAGCCTC TGCACAACTT CCTGACCCAG
961 CTCCAGCCCT TCCTGAAGCT CAAGCAGAAG CAGGCCAACC TCGTGCTGAA GATCATCGAG
1021 CAGCTGCCCT CCGCCAAGGA ATCCCCGGAC AAGTTCCTGG AGGTGTGCAC CTGGGTGGAC
1081 CAGATCGCCG CTCTGAACGA CTCCAAGACC CGCAAGACCA CTTCCGAAAC CGTCCGCGCC
1141 GTTCTAGACA GTCTCTCCGA GAAGAAGAAG TCGTCCCCCT AAACAGTCTC TCCGAGAAGA
1201 AGAAGTCGTC CCCCTAGCGG CCGCTTCGAG CAGACATGAT AAGATACATT GATGAGTTTG
1261 GACAAACCAC AACTAGAATG CAGTGAAAAA AATGCTTTAT TTGTGAAATT TGTGATGCTA
1321 TTGCTTTATT TGTAACCATT ATAAGCTGCA ATAAACAAGT TAACAACAAC AATTGCATTC
1381 ATTTTATGTT TCAGGTTCAG GGGGAGATGT GGGAGGTTTT TTAAAGCAAG TAAAACCTCT
1441 ACAAATGTGG TAAAATCGAT AAGATCTTGA TCCGGGCTGG CGTAATAGCG AAGAGGCCCG
1501 CACCGATCGC CCTTCCCAAC AGTTGCGCAG CCTGAATGGC GAATGGACGC GCCCTGTAGC
1561 GGCGCATTAA GCGCGGCGGG TGTGGTGGTT ACGCGCAGCG TGACCGCTAC ACTTGCCAGC
1621 GCCCTAGCGC CCGCTCCTTT CGCTTTCTTC CCTTCCTTTC TCGCCACGTT CGCCGGCTTT
 
1681 CCCCGTCAAG CTCTAAATCG GGGGCTCCCT TTAGGGTTCC GATTTAGTGC TTTACGGCAC
1741 CTCGACCCCA AAAAACTTGA TTAGGGTGAT GGTTCACGTA GTGGGCCATC G
SEQ ID NO: 145 (Exon 45 Forward PCR primer)
   1 CTAACCGAGA GGGTGCTTTT TTC
SEQ ID NO: 146 (Exon 45 Reverse PCR primer)
   1 GTGTTTAGGT CAACTAATGT GTTTATTTTG
SEQ ID NO: 147 (MDX-1/2 Meganuclease) 
1 MAPKKKRKVH MNTKYNKEFL LYLAGFVDGD GSIFACIHPS QAYKFKHRLT LHFTVTQKTQ
61 RRWFLDKLVD EIGVGYVQDV GSVSQYRLSQ IKPLHNFLTQ LQPFLKLKQK QANLVLKIIE
121 QLPSAKESPD KFLEVCTWVD QIAALNDSKT RKTTSETVRA VLDSLPGSVG GLSPSQASSA
181 ASSASSSPGS GISEALRAGA GSGTGYNKEF LLYLAGFVDG DGSISATIAP AQYGKFKHYL
241 GLRFYVSQKT QRRWFLDKLV DEIGVGYVSD QGSVSRYCLS QIKPLHNFLT QLQPFLKLKQ
301 KQANLVLKII EQLPSAKESP DKFLEVCTWV DQIAALNDSK TRKTTSETVR AVLDSLSEKK
361 KSSP
SEQ ID NO: 148 (MDX-13/14 Meganuclease) 
1 MAPKKKRKVH MNTKYNKEFL LYLAGFVDGD GSIYACIRPT QSVKFKHDLL LCFDVSQKTQ
61 RRWFLDKLVD EIGVGYVYDR GSVSSYRLSE IKPLHNFLTQ LQPFLKLKQK QANLVLKIIE
121 QLPSAKESPD KFLEVCTWVD QIAALNDSKT RKTTSETVRA VLDSLPGSVG GLSPSQASSA
181 ASSASSSPGS GISEALRAGA GSGTGYNKEF LLYLAGFVDG DGSIWASIEP RQQSKFKHQL
241 RLGFSVYQKT QRRWFLDKLV DEIGVGYVRD TGSVSCYCLS QIKPLHNFLT QLQPFLKLKQ
301 KQANLVLKII EQLPSAKESP DKFLEVCTWV DQIAALNDSK TRKTTSETVR AVLDSLSEKK
361 KSSP
SEQ ID NO: 149 (MDX-1/2 Recognition Sequence)
   1 TTCTGTGATG TGAGGACATA TA
SEQ ID NO: 150 (MDX-13/14 Recognition Sequence)
   1 ACTAATGAAA CACCACTCCA CA
SEQ ID NO: 151 (Mouse *DMD* Intron 22 Forward Primer)
   1 GTCTTATCAG TCAAGAGATC ATATTG
SEQ ID NO: 152 (Mouse *DMD* Intron 23 Reverse Primer)
   1 GTGTCAGTAA TCTCTATCCC TTTCATG
SEQ ID NO: 153 (Mutant Sequence from Mouse *DMD* Gene) 
1 AGAATTTAAA TATTAACAAA CTATAACACT ATGATTAAAT GCTTGATATT GAGTAGTTAT
61 TTTAATAGCC TAAGTCTGGA AATTAAATAC TAGTAAGAGA AACTTCTAGA ATTTAAATAT
121 TAACAAACTA TAACACTATG ATTAAATGCT TGATATTGAG TAGTTATTTT AATAGCCTAA
181 GTCTGGAAAT TAAATACTAG TAAGAGAAAC TTCT
SEQ ID NO: 154 (Mutant Sequence from Mouse *DMD* Gene) 
1 TTTAATAGCC TAAGTCTGGA AATACTCCAC AGGTGATTTC AGCCACTTTA TGAACTGCTG
61 GAAGCAAAAA TGAGATCTTT
SEQ ID NO: 155 (Mutant Sequence from Mouse *DMD* Gene) 1 TTAGTTAGAA TTTAAATATT AACAAACTAT AACACTATGA TTAAATGCTT GATATTGAGT 
 61 AGTTATTTTA ATAGCCTAAG TCTGGAAATT AAATACTAGT AAGAGAAACT TCTGTGATGT
121 GACCACTCCA CAGGTGATTT CAGCCACTTT ATGAACTGCT GGAAGCAAAA ATGAGATCTT
181 T
SEQ ID NO: 156 (Mutant Sequence from Mouse *DMD* Gene) 
1 TATAACACTA TGATTAAATG CTTGATATTG AGTAGTTATT TTATGTGTCA TACCTTCTTG
61 GATTGTCTGT ATAAATGAAT TGATTTTTTT TCACCAACTC CAAGTATACT TAACATTTTA
121 ACATAATAAT TTAAAATATC CTTATTCCAT TATGTTCATT TTTTAAGTTG TAGATATGAT
181 TTAGCTCACA GCATACATAT ATACACATGT ATTACATATG CATATATTAT ATATATGGCA
241 GACATATGTT TTCACTACCA TATTTCACTT TTGAATTATG AATATATGTT TAATTTCTGC
301 CATATTTCCT TCCCTACATT GACTTCTATT AATTTAGTAT TTCAGTAGTT CTAACACATT
361 AATAATAACC TAGACTCAAT ACAGTAATCT AACAATTATA TTTGTGCCTG TAATTCTAAG
421 TTAGTTAAAT TCATAGGTTG TGTTTCTCAT AGTTGGCCAT TTGTGAAATA TAATAATATC
481 CGAAAAGAAA GTTCAAAAAT GTCATGACTT CATATAGAGT TATTGAAACA GTGCCCTTAC
541 TTTCATTCTG GCCATGCTAG TGACTTGATC ATTCTTGTAT TTTACAGCTA AAACACTACC
601 AAAAGTGTCA AATCCATGAT CTACATGTTT GACCACTCCA CAGGTGATTT CAGCCACTTT
661 ATGAACTGCT GGAAGCAAAA ATGAGATCTT T
SEQ ID NO: 157 (Mutant Sequence from Mouse *DMD* Gene) 
1 TTGAGTAGTT ATTTTAATAG CCTAAGTCTG GAAATTAAAT ACTAGTAAGA GAAACTTCTG
61 TGATGTGCAC AGGTGATTTC AGCCACTTTA TGAACTGCTG GAAGCAAAAA TG
SEQ ID NO: 158 (Mutant Sequence from Mouse *DMD* Gene) 
1 GATATTGAGT AGTTATTTTA ATAGCCTAAG TCTGGAAATT AAATACTAGT AGATTTCAGC
61 CACTTTATGA ACTGCTGGAA GCAAAAATGA
SEQ ID NO: 159 (Mutant Sequence from Mouse *DMD* Gene) 
1 AATACTAGTA AGAGAAACTT CTGTGATGTG AGGACTCCAC AGGTGATTTC AGCCACTTTA
61 TGAACTGCTG GAAGCAAAAA TGAGATCTTT GCAACATGAA GCAGTTGCTC AGTTCATTAA
121 ACTGTGTTCA ATATTTCAGC CATAACATAC ATTAGAGAAT GATTTATATT GTTCAAACAT
181 TT
SEQ ID NO: 160 (Mutant Sequence from Mouse *DMD* Gene) 
1 AATACTAGTA AGAGAAACTT CTGTGATGTG AGGACATTTC AGCCACTTTA TGAACTGCTG
61 GAAGCAAAAA TGAGATCTTT GCAACATGAA GCAGTTGCTC AGTTCATTAA ACTGTGTTCA
121 ATATTTCAGC CATAACATAC ATTAGAGAAT GATTTATATT GTTCAAACAT TT
SEQ ID NO: 161 (Mutant Sequence from Mouse *DMD* Gene) 
  1 AATACTAGTA AGAGAAGATT TCAGCCACTT TATGAACTGC TGGAAGCAAA AATGAGATCT
 61 TTGCAACATG AAGCAGTTGC TCAGTTCATT AAACTGTGTT CAATATTTCA GCCATAACAT
121 ACATTAGAGA ATGATTTATA TTGTTCAAAC ATTT
SEQ ID NO: 162 (Mutant Sequence from Mouse *DMD* Gene) 
1 TTTAATAGCC TAAGTCTGGA AATTAAATAC TAGTAAGAGA GTGATTTCAG CCACTTTATG
61 AACTGCTGGA AGCAAAAATG A
SEQ ID NO: 163 (Mutant Sequence from Mouse *DMD* Gene) 
1 TTAGTTAGAA TTTAAATATT AACAAACTAT AACACTATGA TTAAATGCTT GATATTGAGT
61 AGTTATTTTA ATAGCCTAAG TCTGGAAATT AAATACTAGT TCAGCCACTT TATGAACTGC
121 TGGAAGCAAA AATGAGATCT CATTAAACTG TGTTCAATAT TTCAGCCATA ACATACATTA
181 GAGAATGATT TATATTGTTC AAACATTTGG TGCTCTATTT TTGCATGACG TGGGA
SEQ ID NO: 164 (Mutant Sequence from Mouse *DMD* Gene) 
1 TTAGTTAGAA TTTAAATATT AACAAACTAT AACACTATGA TTAAATGCTT GATATTGAGT
61 AGTTATTTTA ATAGCCTAAG TCTGGAAATT AAATACTAGT AAGAGAAACT TCTGTGATGT
121 GAGGACATAT AAAGACTAAT TTTTTTGTTG ATTCTAAAAA TCCACAGGTG ATTTCAGCCA
181 CTTTATGAAC TGCTGGAAGC AAAAATGAGA TCTTTGCAAC ATGAAGCAGT TGCTCAGTTC
241 ATTAAACTGT GTTCAATATT TCAGCCATAA CATACATTAG AGAATGATTT ATATTGTTCA
301 AACATTTGGT GCTCTATTTT TGCATGACGT GGGA
SEQ ID NO: 165 (Mutant Sequence from Mouse *DMD* Gene) 
1 TTAGTTAGAA TTTAAATATT AACAAACTAT AACACTATGA TTAAATGCTT GATATTGAGT
61 AGTTATTTTA ATAGCCTAAG TCTGGAAATT AAATACTAGT AAGAGAAACT TCTGTGATGT
121 GAGGACATAT AAAGACTAAT TTTTTCACTC CACAGGTGAT TTCAGCCACT TTATGAACTG
181 CTGGAAGCAA AAATGAGATC TTT
SEQ ID NO: 166 (Mutant Sequence from Mouse *DMD* Gene) 
1 TTATTTTAAT AGCCTAAGTC TGGAAATTAA ATACTAGTAA GAGAAACTTC TGTGATGTGA
61 GGACATATAA AGACTAATTT TTTTGTTGAT TCTAAAAATC CCATGTTGTA TACTTATTCT
121 TTTTAAATCT GAAAATATAT TAATCATATA TTGCCTAAAT GTCTTAATAA TGTTTCACTG
181 TAGGTAAGTT AAAATGTATC ACATATATAA TAAACATAGT TATTAATGCA TAGATATTCA
241 GTAAAATTAT GACTTCTAAA TTTCTGTCTA AATATAATAT GCCCTGTAAT ATAATAGAAA
301 TTATTCATAA GAATACATAT ATATTGCTTT ATCAGATATT CTACTTTGTT TAGATCTCTA
361 AATTACATAA ACTTTTATTT ACCTTCTTCT TGATATGAAT GAAACTCATC AAATATGCGT
421 GTTAGTGTAA ATGAACTTCT ATTTAAACTC CACAGGTGAT TTCAGCCACT TTATGAAC
SEQ ID NO: 167 (Mutant Sequence from Mouse *DMD* Gene) 
1 TTAGTTAGAA TTTAAATATT AACAAACTAT AACACTATGA TTAAATGCTT GATATTGAGT
61 AGTTATTTTA ATAGCCTAAG TCTGGAAATT AAATACTAGT AAGAGAAACT TCTGTGATGT
121 GAGGACATAT AAAGACTAAT TTTTTTGTTG ATTCTAAAAA TCCCATGTTG TATACTTATT
181 CTTTTTAAAT CTGAAAATAT ATTAATCATA TATTGCCTAA ATGTCTTAAT AATGTTTCAC
241 TGTAGGTAAG TTAAAATGTA TCACATATAT AATAAACATA GTTATTAATG CATAGATATT
301 CAGTAAAATT ATGACTTCTA AATTTCTGTC TAAATATAAT ATGCCCTGTA ATATAATAGA
361 AATTATTCAT AAGAATACAT ATATATTGCT TTATCAGATA TTCTACTTTG TTTAGATCTC
421 TAAATTACAT AAACTTTTAT TTACCTTCTT CTTGATATGA ATGAAACTCA TCAAATATGC
481 GTGTTAGTGT AAATGAACTT CTATTTAATT TTGAGGCTCT GCAAAGTTCT CCACAGGTGA
541 TTTCAGCCAC TTTATGAACT GCTGGAAGCA AAAATGAGAT CTTTGCAACA TGAAGCAGTT
601 GCTCAGTTCA TTAAACTGTG TTCAATATTT CAGCCATAAC ATACATTAGA GAATGATTTA
661 TATTGTTCAA ACATTTGGTG CTCTATTTTT GCATGACGTG GGA
SEQ ID NO: 168 (Mutant Sequence from Mouse *DMD* Gene) 
1 AATACTAGTA AGAGAAACTT CTGTGATGTG AGGACATATA AAGACTAATT TTTTTGTTGA
61 TTCTAAAAAT CCCATGTTGT ATACTTATTC TTTTTAAATC TGAAAATATA TTAATCATAT
121 ATTGCCTAAA TGTCTTAATA ATGTTTCACT GTAGGTAAGT TAAAATGTAT CACATATATA
181 ATAAACATAG TTATTAATGC ATAGATATTC AGTAAAATTA TGACTTCTAA ATTTCTGTCT
241 AAATATAATA TGCCCTGTAA TATAATAGAA ATTATTCATA AGAATACATA TATATTGCTT
301 TATCAGATAT TCTACTTTGT TTAGATCTCT AAATTACATA AACTTTTATT TACCTTCTTC
361 TTGATATGAA TGAAACTCAT CAAATATGCG TGTTAGTGTA AATGAACTTC TATTTAATTT
 
421 TGAGGCTCTG CAAAGTTCTT TGAAAGAGCA ACAAAATGGC TTCACCACTC CACAGGTGAT
481 TTCAGCCACT TTATGAACTG CTGGAAGCAA AAATGAGATC TTTGCAACAT GAAGCAGTTG
541 CTCAGTTCAT TAAACTGTGT TCAATATTTC AGCCATAACA TACATTAGAG AATGATTTAT
601 ATTGTTCAAA CATTT
SEQ ID NO: 169 (Mutant Sequence from Mouse *DMD* Gene) 
1 TTAGTTAGAA TTTAAATATT AACAAACTAT AACACTATGA TTAAATGCTT GATATTGAGT
61 AGTTATTTTA ATAGCCTAAG TCTGGAAATT AAATACTAGT AAGAGAAACT TCTGTGATGT
121 GAGGACATAT AAAGACTAAT TTTTTTGTTG ATTCTAAAAA TCCCATGTTG TATACTTATT
181 CTTTTTAAAT CTGAAAATAT ATTAATCATA TATTGCCTAA ATGTCTTAAT AATGTTTCAC
241 TGTAGGTAAG TTAAAATGTA TCACATATAT AATAAACATA GTTATTAATG CATAGATATT
301 CAGTAAAATT ATGACTTCTA AATTTCTGTC TAAATATAAT ATGCCCTGTA ATATAATAGA
361 AATTATTCAT AAGAATACAT ATATATTGCT TTATCAGATA TTCTACTTTG TTTAGATCTC
421 TAAATTACAT AAACTTTTAT TTACCTTCTT CTTGATATGA ATGAAACTCA TCAAATATGC
481 GTGTTAGTGT AAATGAACTT CTATTTAATT TTGAGGCTCT GCAAAGTTCT TTGAAAGAGC
541 AACAAAATGG CTTCAACTAT CTGAGTGACA CTGTGAAGGA GATGGCCAAG AAAGCACCTT
601 CAGAAATATG CCATTTCAGC CACTTTATGA ACTGCTGGAA GCAAAAATGA GATCTTTGCA
661 ACATGAAGCA GTTGCTCAGT TCATTAAACT GTGTTCAATA TTTCAGCCAT AACATACATT
721 AGAGAATGAT TTATATTGTT CAAACATTTG GTGCTCTATT TTTGCATGAC GTGGGA
SEQ ID NO: 170 (Mutant Sequence from Mouse *DMD* Gene) 
1 GTCTGGAAAT TAAATACTAG TAAGAGAAAC TTCTGTGATG TGAGGACATA TAAAGACTAA
61 TTTTTTTGTT GATTCTAAAA ATCCCATGTT GTATACTTAT TCTTTTTAAA TCTGAAAATA
121 TATTAATCAT ATATTGCCTA AATGTCTTAA TAATGTTTCA CTGTAGGTAA GTTAAAATGT
181 ATCACATATA TAATAAACAT AGTTATTAAT GCATAGATAT TCAGTAAAAT TATGACTTCT
241 AAATTTCTGT CTAAATATAA TATGCCCTGT AATATAATAG AAATTATTCA TAAGAATACA
301 TATATATTGC TTTATCAGAT ATTCTACTTT GTTTAGATCT CTAAATTACA TAAACTTTTA
361 TTTACCTTCT TCTTGATATG AATGAAACTC ATCAAATATG CGTGTTAGTG TAAATGAACT
421 TCTATTTAAT TTTGAGGCTC TGCAAAGTTC TTTGAAAGAG CAACAAAATG GCTTCAACTA
481 TCTGAGTGAC ACTGTGAAGG AGATGGCCAA GAAAGCACCT TCAGAAATAT GCCAGAAATA
541 TCTGTCAGAA TTTGAAGAGA TTGAGGGGCA CTGGAAGAAA CTTTCCTCCC AGTTGGTGGA
601 AAACACCACT CCACAGGTGA TTTCAGCCAC TTTAT
SEQ ID NO: 171 (Mutant Sequence from Mouse *DMD* Gene) 
1 TGGAAATTAA ATACTAGTAA GAGAAACTTC TGTGATGTGA GGACATATAA AGACTAATTT
61 TTTTGTTGAT TCTAAAAATC CCATGTTGTA TACTTATTCT TTTTAAATCT GAAAATATAT
121 TAATCATATA TTGCCTAAAT GTCTTAATAA TGTTTCACTG TAGGTAAGTT AAAATGTATC
181 ACATATATAA TAAACATAGT TATTAATGCA TAGATATTCA GTAAAATTAT GACTTCTAAA
241 TTTCTGTCTA AATATAATAT GCCCTGTAAT ATAATAGAAA TTATTCATAA GAATACATAT
301 ATATTGCTTT ATCAGATATT CTACTTTGTT TAGATCTCTA AATTACATAA ACTTTTATTT
361 ACCTTCTTCT TGATATGAAT GAAACTCATC AAATATGCGT GTTAGTGTAA ATGAACTTCT
421 ATTTAATTTT GAGGCTCTGC AAAGTTCTTT GAAAGAGCAA CAAAATGGCT TCAACTATCT
481 GAGTGACACT GTGAAGGAGA TGGCCAAGAA AGCACCTTCA GAAATATGCC AGAAATATCT
541 GTCAGAATTT GAAGAGATTG AGGGGCACTG GAAGAAACTT TCCTCCCAGT TGGTGGAAAG
601 CTGCCAAAAG CTAGAAGAAC ATATGAATAA ACTTCGAAAA TTTCAGGTAA GCCGAGGTTT
661 GGCCTTTAAA CTATATTTTT CCACTCCACA GGTGATTTCA GCCACTTTAT GAAC
SEQ ID NO: 172 (Mutant Sequence from Mouse *DMD* Gene) 
1 CCTAAGTCTG GAAATTAAAT ACTAGTAAGA GAAACTTCTG TGATGTGAGG ACATATAAAG
61 ACTAATTTTT TTGTTGATTC TAAAAATCCC ATGTTGTATA CTTATTCTTT TTAAATCTGA
121 AAATATATTA ATCATATATT GCCTAAATGT CTTAATAATG TTTCACTGTA GGTAAGTTAA
181 AATGTATCAC ATATATAATA AACATAGTTA TTAATGCATA GATATTCAGT AAAATTATGA
241 CTTCTAAATT TCTGTCTAAA TATAATATGC CCTGTAATAT AATAGAAATT ATTCATAAGA
 
 301 ATACATATAT ATTGCTTTAT CAGATATTCT ACTTTGTTTA GATCTCTAAA TTACATAAAC
 361 TTTTATTTAC CTTCTTCTTG ATATGAATGA AACTCATCAA ATATGCGTGT TAGTGTAAAT
 421 GAACTTCTAT TTAATTTTGA GGCTCTGCAA AGTTCTTTGA AAGAGCAACA AAATGGCTTC
 481 AACTATCTGA GTGACACTGT GAAGGAGATG GCCAAGAAAG CACCTTCAGA AATATGCCAG
 541 AAATATCTGT CAGAATTTGA AGAGATTGAG GGGCACTGGA AGAAACTTTC CTCCCAGTTG
 601 GTGGAAAGCT GCCAAAAGCT AGAAGAACAT ATGAATAAAC TTCGAAAATT TCAGGTAAGC
 661 CGAGGTTTGG CCTTTAAACT ATATTTTTTC ACATAGCAAT TAATTGGAAA ATGTGATGGG
 721 AAACAGATAT TTTACCCAGA GTCCTTCAAA GATATTGATG ATATCAAAAG CCAAATCTAT
 781 TTCAAAGGAT TGCAACTTGC CTATTTTTCC TATGAAAACA GTAATGTGTC ATACCTTCTT
 841 GGATTGTCTG TATAAATGAA TTGATTTTTT TTCACCAACT CCAAGTATAC TTAACATTTT
 901 AACATAATAA TTTAAAATAT CCTTATTCCA TTATGTTCAT TTTTTAAGTT GTAGATATGA
 961 TTTAGCTCAC AGCATACATA TATACACATG TATTACATAT GCATATATTA TATATATGGC
1021 AGACATATGT TTTCACTACC ATATTTCACT TTTGAATTAT GAATATATGT TTAATTTCTG
1081 CCATATTTCC TTCCCTACAT TGACTTCTAT TAATTTAGTA TTTCAGTAGT TCTAACACAT
1141 TAATAATAAC CTAGACTCAA TACAGTAATC TAACAATTAT ATTTGTGCCT GTAATTCTAA
1201 GTTAGTTAAA TTCATAGGTT GTGTTTCTCA TAGTTGGCCA TTTGTGAAAT ATAATAATAT
1261 CCGAAAAGAA AGTTCAAAAA TGTCATGACT TCATATAGAC AGGTGATTTC AGCCACTTTA
1321 TG
SEQ ID NO: 173 (pAAV-MDX Plasmid) 
1 GGGGGGGGGG GGGGGGGTTG GCCACTCCCT CTCTGCGCGC TCGCTCGCTC ACTGAGGCCG
61 GGCGACCAAA GGTCGCCCGA CGCCCGGGCT TTGCCCGGGC GGCCTCAGTG AGCGAGCGAG
121 CGCGCAGAGA GGGAGTGGCC AACTCCATCA CTAGGGGTTC CTAGATCTTC AATATTGGGT
181 ATTAGTCATC GCTATTACCA TGATGATGCG GTTTTGGCAG TACACCAATG GGCGTGGATA
241 GCGGTTTGAC TCACGGGGAT TTCCAAGTCT CCACCCCATT GACGTCAATG GGAGTTTGTT
301 TTGGCACCAA AATCAACGGG ACTTTCCAAA ATGTCGTAAT AACCCCGCCC CGTTGACGCA
361 AATGGGCGGT AGGCGTGTAC GGTGGGAGGT CTATATAAGC AGAGCTCGTT TAGTGAACCG
421 TCAGATCACT AGAAGCTTTA TTGCGGTAGT TTATCACAGT TAAATTGCTA GCGCAGTCAG
481 TGCTTCTGAC ACAACAGTCT CGAACTTAAG CTGCAGAAGT TGGTCGTGAG GCACTGGGCA
541 GGTAAGTATC AAGGTTACAA GACAGGTTTA AGGACACCAA TAGAAACTGG GCTTGTCGAG
601 ACAGAGAAGA CTCTTGCGTT TCTGATAGGC ACCTATTGGT CTTACTGACA TCCACTTTGC
661 CTTTCTCTCC ACAGGTAATT GTGAGCGGAT AACAATTGAT GTCGCACAGG CCACGGATTA
721 GGCACCCCAG GCTTGACACT TTATGCTTCC GGCTCGTATA TTGTGTGGAA TTGTGAGCGG
781 ATAACAATTT CACACAGGAG ATATATATAT GGGCTAGGCC ACCATGGCAC CGAAGAAGAA
841 GCGCAAGGTG CATATGAATA CAAAATATAA TAAAGAGTTC TTACTCTACT TAGCAGGGTT
901 TGTAGACGGT GACGGTTCCA TCTTTGCCTG TATCCATCCT AGTCAAGCGT ATAAGTTCAA
961 GCACCGGCTG ACTCTCCATT TCACGGTCAC TCAGAAGACA CAGCGCCGTT GGTTCCTCGA
1021 CAAGCTGGTG GACGAGATCG GTGTGGGTTA CGTGCAGGAC GTGGGCAGCG TCTCCCAGTA
1081 CCGGCTGTCC CAGATCAAGC CTTTGCATAA TTTTTTAACA CAACTACAAC CTTTTCTAAA
1141 ACTAAAACAA AAACAAGCAA ATTTAGTTTT AAAAATTATT GAACAACTTC CGTCAGCAAA
1201 AGAATCCCCG GACAAATTCT TAGAAGTTTG TACATGGGTG GATCAAATTG CAGCTCTGAA
1261 TGATTCGAAG ACGCGTAAAA CAACTTCTGA AACCGTTCGT GCTGTGCTAG ACAGTTTACC
1321 AGGATCCGTG GGAGGTCTAT CGCCATCTCA GGCATCCAGC GCCGCATCCT CGGCTTCCTC
1381 AAGCCCGGGT TCAGGGATCT CCGAAGCACT CAGAGCTGGA GCAGGTTCCG GCACTGGATA
1441 CAACAAGGAA TTCCTGCTCT ACCTGGCGGG CTTCGTCGAC GGGGACGGCT CCATCTCTGC
1501 CACTATCGCT CCGGCTCAGT ATGGTAAGTT CAAGCACTAT CTGGGGCTCC GGTTCTATGT
1561 CAGTCAGAAG ACACAGCGCC GTTGGTTCCT CGACAAGCTG GTGGACGAGA TCGGTGTGGG
1621 TTACGTGAGT GACCAGGGCA GCGTCTCCAG GTACTGTCTG TCCCAGATCA AGCCTCTGCA
1681 CAACTTCCTG ACCCAGCTCC AGCCCTTCCT GAAGCTCAAG CAGAAGCAGG CCAACCTCGT
1741 GCTGAAGATC ATCGAGCAGC TGCCCTCCGC CAAGGAATCC CCGGACAAGT TCCTGGAGGT
1801 GTGCACCTGG GTGGACCAGA TCGCCGCTCT GAACGACTCC AAGACCCGCA AGACCACTTC
1861 CGAAACCGTC CGCGCCGTTC TAGACAGTCT CTCCGAGAAG AAGAAGTCGT CCCCCTAAGG
1921 TACCAGCGGC CGCTTCGAGC AGACATGATA AGATACATTG ATGAGTTTGG ACAAACCACA
1981 ACTAGAATGC AGTGAAAAAA ATGCTTTATT TGTGAAATTT GTGATGCTAT TGCTTTATTT
2041 GTAACCATTA TAAGCTGCAA TAAACAAGTT GTATTAGTCA TCGCTATTAC CATGATGATG
2101 CGGTTTTGGC AGTACACCAA TGGGCGTGGA TAGCGGTTTG ACTCACGGGG ATTTCCAAGT
2161 CTCCACCCCA TTGACGTCAA TGGGAGTTTG TTTTGGCACC AAAATCAACG GGACTTTCCA
2221 AAATGTCGTA ATAACCCCGC CCCGTTGACG CAAATGGGCG GTAGGCGTGT ACGGTGGGAG
 
2281 GTCTATATAA GCAGAGCTCG TTTAGTGAAC CGTCAGATCA CTAGAAGCTT TATTGCGGTA
2341 GTTTATCACA GTTAAATTGC TAGCGCAGTC AGTGCTTCTG ACACAACAGT CTCGAACTTA
2401 AGCTGCAGAA GTTGGTCGTG AGGCACTGGG CAGGTAAGTA TCAAGGTTAC AAGACAGGTT
2461 TAAGGACACC AATAGAAACT GGGCTTGTCG AGACAGAGAA GACTCTTGCG TTTCTGATAG
2521 GCACCTATTG GTCTTACTGA CATCCACTTT GCCTTTCTCT CCACAGGTAA TTGTGAGCGG
2581 ATAACAATTG ATGTCGCACA GGCCACGGAT TAGGCACCCC AGGCTTGACA CTTTATGCTT
2641 CCGGCTCGTA TATTGTGTGG AATTGTGAGC GGATAACAAT TTCACACAGG AGATATATAT
2701 ATGGGCTAGG CCACCATGGC ACCGAAGAAG AAGCGCAAGG TGCATATGAA TACAAAATAT
2761 AATAAAGAGT TCTTACTCTA CTTAGCAGGG TTTGTAGACG GTGACGGTTC CATCTATGCC
2821 TGTATCAGGC CGACGCAGAG TGTGAAGTTC AAGCACGATC TGCTGCTCTG TTTCGATGTC
2881 TCTCAGAAGA CACAGCGCCG TTGGTTCCTC GACAAGCTGG TGGACGAGAT CGGTGTGGGT
2941 TACGTGTATG ACCGTGGCAG CGTCTCCTCG TACAGGCTGT CCGAGATCAA GCCTTTGCAT
3001 AATTTTTTAA CACAACTACA ACCTTTTCTA AAACTAAAAC AAAAACAAGC AAATTTAGTT
3061 TTAAAAATTA TTGAACAACT TCCGTCAGCA AAAGAATCCC CGGACAAATT CTTAGAAGTT
3121 TGTACATGGG TGGATCAAAT TGCAGCTCTG AATGATTCGA AGACGCGTAA AACAACTTCT
3181 GAAACCGTTC GTGCTGTGCT AGACAGTTTA CCAGGATCCG TGGGAGGTCT ATCGCCATCT
3241 CAGGCATCCA GCGCCGCATC CTCGGCTTCC TCAAGCCCGG GTTCAGGGAT CTCCGAAGCA
3301 CTCAGAGCTG GAGCAGGTTC CGGCACTGGA TACAACAAGG AATTCCTGCT CTACCTGGCG
3361 GGCTTCGTCG ACGGGGACGG CTCCATCTGG GCCTCGATCG AGCCTAGGCA ACAGTCTAAG
3421 TTCAAGCACC AGCTGCGGCT CGGGTTCTCG GTCTATCAGA AGACACAGCG CCGTTGGTTC
3481 CTCGACAAGC TGGTGGACGA GATCGGTGTG GGTTACGTGC GTGACACTGG CAGCGTCTCC
3541 TGTTACTGTC TGTCCCAGAT CAAGCCTCTG CACAACTTCC TGACCCAGCT CCAGCCCTTC
3601 CTGAAGCTCA AGCAGAAGCA GGCCAACCTC GTGCTGAAGA TCATCGAGCA GCTGCCCTCC
3661 GCCAAGGAAT CCCCGGACAA GTTCCTGGAG GTGTGCACCT GGGTGGACCA GATCGCCGCT
3721 CTGAACGACT CCAAGACCCG CAAGACCACT TCCGAAACCG TCCGCGCCGT TCTAGACAGT
3781 CTCTCCGAGA AGAAGAAGTC GTCCCCCTAA GGTACCAGCG GCCGCTTCGA GCAGACATGA
3841 TAAGATACAT TGATGAGTTT GGACAAACCA CAACTAGAAT GCAGTGAAAA AAATGCTTTA
3901 TTTGTGAAAT TTGTGATGCT ATTGCTTTAT TTGTAACCAT TATAAGCTGC AATAAACAAG
3961 TTAACAACAA CAATTGCATT CATTTTATGT TTCAGGTTCA GGGGGAGATG TGGGAGGTTT
4021 TTTAAAGCAA GTAAAACCTC TACAAATGTG GTAAAATCGA TAAGGATCTA GGAACCCCTA
4081 GTGATGGAGT TGGCCACTCC CTCTCTGCGC GCTCGCTCGC TCACTGAGGC CGCCCGGGCA
4141 AAGCCCGGGC GTCGGGCGAC CTTTGGTCGC CCGGCCTCAG TGAGCGAGCG AGCGCGCAGA
4201 GAGGGAGTGG CCAACCCCCC CCCCCCCCCC CCTGCAGCCT GGCGTAATAG CGAAGAGGCC
4261 CGCACCGATC GCCCTTCCCA ACAGTTGCGT AGCCTGAATG GCGAATGGCG CGACGCGCCC
4321 TGTAGCGGCG CATTAAGCGC GGCGGGTGTG GTGGTTACGC GCAGCGTGAC CGCTACACTT
4381 GCCAGCGCCC TAGCGCCCGC TCCTTTCGCT TTCTTCCCTT CCTTTCTCGC CACGTTCGCC
4441 GGCTTTCCCC GTCAAGCTCT AAATCGGGGG CTCCCTTTAG GGTTCCGATT TAGTGCTTTA
4501 CGGCACCTCG ACCCCAAAAA ACTTGATTAG GGTGATGGTT CACGTAGTGG GCCATCGCCC
4561 TGATAGACGG TTTTTCGCCC TTTGACGTTG GAGTCCACGT TCTTTAATAG TGGACTCTTG
4621 TTCCAAACTG GAACAACACT CAACCCTATC TCGGTCTATT CTTTTGATTT ATAAGGGATT
4681 TTGCCGATTT CGGCCTATTG GTTAAAAAAT GAGCTGATTT AACAAAAATT TAACGCGAAT
4741 TTTAACAAAA TATTAACGTT TACAATTTCC TGATGCGCTA TTTTCTCCTT ACGCATCTGT
4801 GCGGTATTTC ACACCGCATA TGGTGCACTC TCAGTACAAT CTGCTCTGAT GCCGCATAGT
4861 TAAGCCAGCC CCGACACCCG CCAACACCCG CTGACGCGCC CTGACGGGCT TGTCTGCTCC
4921 CGGCATCCGC TTACAGACAA GCTGTGACCG TCTCCGGGAG CTGCATGTGT CAGAGGTTTT
4981 CACCGTCATC ACCGAAACGC GCGAGACGAA AGGGCCTCGT GATACGCCTA TTTTTATAGG
5041 TTAATGTCAT GATAATAATG GTTTCTTAGA CGTCAGGTGG CACTTTTCGG GGAAATGTGC
5101 GCGGAACCCC TATTTGTTTA TTTTTCTAAA TACTTTCAAA TATGTATCCG CTCATGAGAC
5161 AATAACCCTG ATAAATGCTT CAATAATATT GAAAAAGGAA GAGTATGAGT ATTCAACATT
5221 TCCGTGTCGC CCTTATTCCC TTTTTTGCGG CATTTTGCCT TCCTGTTTTT GCTCACCCAG
5281 AAACGCTGGT GAAAGTAAAA GATGCTGAAG ATCAGTTGGG TGCACGAGTG GGTTACATCG
5341 AACTGGATCT CAACAGCGGT AAGATCCTTG AGAGTTTTCG CCCCGAAGAA CGTTTTCCAA
5401 TGATGAGCAC TTTTAAAGTT CTGCTATGTG GCGCGGTATT ATCCCGTATT GACGCCGGGC
5461 AAGAGCAACT CGGTCGCCGC ATACACTATT CTCAGAATGA CTTGGTTGAG TACTCACCAG
5521 TCACAGAAAA GCATCTTACG GATGGCATGA CAGTAAGAGA ATTATGCAGT GCTGCCATAA
5581 CCATGAGTGA TAACACTGCG GCCAACTTAC TTCTGACAAC GATCGGAGGA CCGAAGGAGC
5641 TAACCGCTTT TTTGCACAAC ATGGGGGATC ATGTAACTCG CCTTGATCGT TGGGAACCGG
5701 AGCTGAATGA AGCCATACCA AACGACGAGC GTGACACCAC GATGCCTGTA GCAATGGCAA
5761 CAACGTTGCG CAAACTATTA ACTGGCGAAC TACTTACTCT AGCTTCCCGG CAACAATTAA
 
5821 TAGACTGGAT GGAGGCGGAT AAAGTTGCAG GACCACTTCT GCGCTCGGCC CTTCCGGCTG
5881 GCTGGTTTAT TGCGGATAAA TCTGGAGCCG GTGAGCGTGG GTCTCGCGGT ATCATTGCAG
5941 CACTGGGGCC AGATGGTAAG CCCTCCCGTA TCGTAGTTAT CTACACGACG GGGAGTCAGG
6001 CAACTATGGA TGAACGAAAT AGACAGATCG CTGAGATAGG TGCCTCACTG ATTAAGCATT
6061 GGTAACTGTC AGACCAAGTT TACTCATATA TACTTTAGAT TGATTTAAAA CTTCATTTTT
6121 AATTTAAAAG GATCTAGGTG AAGATCCTTT TTGATAATCT CATGACCAAA ATCCCTTAAC
6181 GTGAGTTTTC GTTCCACTGA GCGTCAGACC CCGTAGAAAA GATCAAAGGA TCTTCTTGAG
6241 ATCCTTTTTT TCTGCGCGTA ATCTGCTGCT TGCAAACAAA AAAACCACCG CTACCAGCGG
6301 TGGTTTGTTT GCCGGATCAA GAGCTACCAA CTCTTTTTCC GAAGGTAACT GGCTTCAGCA
6361 GAGCGCAGAT ACCAAATACT GTCCTTCTAG TGTAGCCGTA GTTAGGCCAC CACTTCAAGA
6421 ACTCTGTAGC ACCGCCTACA TACCTCGCTC TGCTAATCCT GTTACCAGTG GCTGCTGCCA
6481 GTGGCGATAA GTCGTGTCTT ACCGGGTTGG ACTCAAGACG ATAGTTACCG GATAAGGCGC
6541 AGCGGTCGGG CTGAACGGGG GGTTCGTGCA CACAGCCCAG CTTGGAGCGA ACGACCTACA
6601 CCGAACTGAG ATACCTACAG CGTGAGCATT GAGAAAGCGC CACGCTTCCC GAAGGGAGAA
6661 AGGCGGACAG GTATCCGGTA AGCGGCAGGG TCGGAACAGG AGAGCGCACG AGGGAGCTTC
6721 CAGGGGGAAA CGCCTGGTAT CTTTATAGTC CTGTCGGGTT TCGCCACCTC TGACTTGAGC
6781 GTCGATTTTT GTGATGCTCG TCAGGGGGGC GGAGCCTATG GAAAAACGCC AGCAACGCGG
6841 CCTTTTTACG GTTCCTGGCC TTTTGCTGGC CTTTTGCTCA CATGTTCTTT CCTGCGTTAT
6901 CCCCTGATTC TGTGGATAAC CGTATTACCG CCTTTGAGTG AGCTGATA
SEQ ID NO: 174 (Mouse *DMD* Intron 22 Forward Primer)
   1 CATTTCATAT TTAGTGACAT AAGATATGAA GTATG
SEQ ID NO: 175 (Mouse *DMD* Intron 23 Reverse Primer)
   1 GTGTCAGTAA TCTCTATCCC TTTCATG
SEQ ID NO: 176 (Mutant Sequence from Mouse *DMD* Gene) 
1 CATTTCATAT TTAGTGACAT AAGATATGAA GTATGATTAT TCAGCCACTT TATGAACTGC
61 TGGAAGCAAA AATGAGATCT TTGCAACATG AAGCAGTTGC TCAGTTCATT AAACTGTGTT
121 CAATATTTCA GCCATAACAT ACATTAGAGA ATGATTTATA TTGTTCAAAC ATTTGGTGCT
181 CTATTTTTGC ATGACGTGGG ATTAAACACA GCACCAACAA TCAAACAATT GCAAAGATGT
241 ATTACAAGTA TTTTTTCTTT TTAAAACAGG AAAGTATACT TATATTTCCA TTGTCCAAAC
301 CATCATGAAA GGGATAGAGA TTACTGACAC
SEQ ID NO: 177 (Mutant Sequence from Mouse *DMD* Gene) 
1 CATTTCATAT TTAGTGACAT AAGATATGAA GTATGATTAT TAAAATTAAA TCACATTATT
61 TTATTATAAT TACTTTACTC CACAGGTGAT TTCAGCCACT TTATGAACTG CTGGAAGCAA
121 AAATGAGATC TTTGCAACAT GAAGCAGTTG CTCAGTTCAT TAAACTGTGT TCAATATTTC
181 AGCCATAACA TACATTAGAG AATGATTTAT ATTGTTCAAA CATTTGGTGC TCTATTTTTG
241 CATGACGTGG GATTAAACAC AGCACCAACA ATCAAACAAT TGCAAAGATG TATTACAAGT
301 ATTTTTTCTT TTTAAAACAG GAAAGTATAC TTATATTTCC ATTGTCCAAA CCATCATGAA
361 AGGGATAGAG ATTACTGACA C
SEQ ID NO: 178 (Mutant Sequence from Mouse *DMD* Gene)

1 CATTTCATAT TTAGTGACAT AAGATATGAA GTATGATTAT TAAAATTAAA TCACATTATT
61 TTATTATAAT TACTTTACAC AGGTGATTTC AGCCACTTTA TGAACTGCTG GAAGCAAAAA
121 TGAGATCTTT GCAACATGAA GCAGTTGCTC AGTTCATTAA ACTGTGTTCA ATATTTCAGC
181 CATAACATAC ATTAGAGAAT GATTTATATT GTTCAAACAT TTGGTGCTCT ATTTTTGCAT
241 GACGTGGGAT TAAACACAGC ACCAACAATC AAACAATTGC AAAGATGTAT TACAAGTATT
301 TTTTCTTTTT AAAACAGGAA AGTATACTTA TATTTCCATT GTCCAAACCA TCATGAAAGG
361 GATAGAGATT ACTGACAC

### Disclosed Items

1. A method for treating Duchenne Muscular Dystrophy in a subject in need thereof, the method comprising contacting the DNA of a muscle cell of the subject with a first nuclease that cuts a first recognition sequence and a second nuclease that cuts a second recognition sequence;
   wherein said first recognition sequence is upstream of a first exon in the dystrophin gene;
   wherein said second recognition sequence is downstream of the first exon in the dystrophin gene; and
   wherein at least one second exon is removed from dystrophin gene in the cell.
2. The method of item 1, wherein each of said first and second nucleases are meganucleases.
3. The method of item 1, wherein each of said first and second nucleases are CRISPRs.
4. The method of item 1, wherein each of said first and second nucleases are compact TALENs.
5. The method of item 1, wherein said first exon is Exon 44.
6. The method of item 1, wherein said first exon is Exon 45.
7. The method of item 1, wherein said first exon is Exon 51.
8. The method of item 5, wherein said first recognition sequence is selected from SEQ ID NOs: 2-28, and wherein said second recognition sequence is selected from SEQ ID NOs: 29-44.
9. The method of item 6, wherein said first recognition sequence is selected from SEQ ID NOs: 45-63, and wherein said second recognition sequence is selected from SEQ ID NOs: 64-74.
10. The method of item 7, wherein said first recognition sequence is selected from SEQ ID NOs: 75-105, and wherein said second recognition sequence is selected from SEQ ID NOs: 106-134.
11. The method of item 8, wherein said first nuclease is SEQ ID NO: 135 and said second nuclease is SEQ ID NO: 136.
12. The method of item 9, wherein said first nuclease is SEQ ID NO: 137 and said second nuclease is SEQ ID NO: 138.
13. The method of any of items 1-12, wherein the genes encoding said first and second nucleases are delivered to the cell using a recombinant adeno-associated virus (AAV).
14. The method of any of items 1-13, wherein said first and second recognition sites are selected to have identical overhangs if cut by the first and second nucleases.
15. A method for removing a DNA sequence from the genome of a cell, the method comprising contacting the DNA with a first nuclease that cuts a first recognition site and a second nuclease that cuts a second recognition site, wherein said first and second recognition sites are selected to have identical overhangs if cut by the first and second nucleases.
16. The method of item 15 wherein, the genes encoding said first and second nucleases are delivered to the cell using a recombinant adeno-associated virus (AAV).
17. The method of item 15 or 16, wherein said first and second nucleases are meganucleases.
18. The method of item 15 or 16, wherein said first and second nucleases are CRISPRs.
19. The method of item 15 or 16, wherein said first and second nucleases are compact TALENs.

## Claims

1. A method for treating Duchenne Muscular Dystrophy in a subject in need thereof, the method comprising contacting the DNA of a muscle cell of the subject with a first nuclease that cuts a first recognition sequence and a second nuclease that cuts a second recognition sequence;
wherein said first recognition sequence is upstream of a first exon in the dystrophin gene;
wherein said second recognition sequence is downstream of the first exon in the dystrophin gene; and
wherein at least one second exon is removed from dystrophin gene in the cell.

2. The method of claim 1, wherein each of said first and second nucleases are meganucleases, each of said first and second nucleases are CRISPRs, or each of said first and second nucleases are compact TALENs.

3. The method of claim 1, wherein said first exon is Exon 44.

4. The method of claim 1, wherein said first exon is Exon 45.

5. The method of claim 1, wherein said first exon is Exon 51.

6. The method of claim 3, wherein said first recognition sequence is selected from SEQ ID NOs: 2-28, and wherein said second recognition sequence is selected from SEQ ID NOs: 29-44.

7. The method of claim 4, wherein said first recognition sequence is selected from SEQ ID NOs: 45-63, and wherein said second recognition sequence is selected from SEQ ID NOs: 64-74.

8. The method of claim 5, wherein said first recognition sequence is selected from SEQ ID NOs: 75-105, and wherein said second recognition sequence is selected from SEQ ID NOs: 106-134.

9. The method of claim 6, wherein said first nuclease is SEQ ID NO: 135 and said second nuclease is SEQ ID NO: 136.

10. The method of claim 7, wherein said first nuclease is SEQ ID NO: 137 and said second nuclease is SEQ ID NO: 138.

11. The method of any of claims 1-10, wherein the genes encoding said first and second nucleases are delivered to the cell using a recombinant adeno-associated virus (AAV).

12. The method of any of claims 1-11, wherein said first and second recognition sites are selected to have identical overhangs if cut by the first and second nucleases.

13. A method for removing a DNA sequence from the genome of a cell, the method comprising contacting the DNA with a first nuclease that cuts a first recognition site and a second nuclease that cuts a second recognition site, wherein said first and second recognition sites are selected to have identical overhangs if cut by the first and second nucleases.

14. The method of claim 13 wherein, the genes encoding said first and second nucleases are delivered to the cell using a recombinant adeno-associated virus (AAV).

15. The method of claim 13 or 14, wherein said first and second nucleases are meganucleases, each of said first and second nucleases are CRISPRs, or each of said first and second nucleases are compact TALENs.
